# EUROPEAN PATENT APPLICATION

(11) **EP 4 023 768 A1**
(43) Date of publication of application: **06.07.2022**
(21) Application number: 20217509.7
(22) Date of filing: 29.12.2020
(51) Int. Cl.: C12Q 1/6883

(54) **METHODS AND KITS FOR DETECTING A RISK FOR DEVELOPING NEUROLOGICAL OR NEUROPHYSIOLOGICAL DISORDERS**

(71) Applicant: Deutsches Zentrum für Neurodegenerative Erkrankungen e.V. (DZNE), 53127 Bonn (DE)
(72) Inventor: ISLAM, Rezaul, 37083 Göttingen (DE); FISCHER, Andre, 37077 Göttingen (DE); SANANBENESI, Farahnaz, 37077 Göttingen (DE)
(74) Representative: Kuttenkeuler, David

(57) **Abstract**

The invention is based on the detection of microRNA (miR) in biological samples derived from subjects for assessing a risk that the subject develops a neurological and / or neuropsychological condition. The invention in particular detects the presence or absence and the amount of microRNA-181a-5p, microRNA146a-5p and/or microRNA148a-3p in such samples, which are shown by the invention to be associated with the neurological and / or neuropsychological condition. In addition, the invention provides a therapeutic application for the treatment of the neurological and / or neuropsychological condition by modulating the expression/function of these miR.

SEQUENCE LISTING

<110> Deutsches Zentrum für Neurodegenerative Erkrankungen e.V.
(DZNE)

<120> METHODS AND KITS FOR DETECTING A RISK FOR DEVELOPING NEUROLOGICAL
OR NEUROPHYSIOLOGICAL DISORDERS

<130> D31771EP

<160> 22

<170> PatentIn version 3.5

<210> 1
<211> 83
<212> RNA
<213> Homo Sapiens

<400> 1
cggggugagg uaguagguug ugugguuuca gggcagugau guugccccuc ggaagauaac 60

uauacaaccu acugccuucc cug 83

<210> 2
<211> 22
<212> RNA
<213> Homo Sapiens

<400> 2
ugagguagua gguugugugg uu 22

<210> 3
<211> 22
<212> RNA
<213> Homo Sapiens

<400> 3
cuauacaacc uacugccuuc cc 22

<210> 4
<211> 82
<212> RNA
<213> Homo Sapiens

<400> 4
ggccugcccg acacucuuuc ccuguugcac uacuauaggc cgcugggaag cagugcaaug 60

augaaagggc aucggucagg uc 82

<210> 5
<211> 21
<212> RNA
<213> Homo Sapiens

<400> 5
acucuuuccc uguugcacua c 21

<210>  6
<211>  22
<212>  RNA
<213>  Homo Sapiens

<400>  6
cagugcaaug augaaagggc au                                                22


<210>  7
<211>  99
<212>  RNA
<213>  Homo Sapiens

<400>  7
ccgaugugua uccucagcuu ugagaacuga auuccauggg uugugucagu gucagaccuc        60

ugaaauucag uucuucagcu gggauaucuc ugucaucgu                               99


<210>  8
<211>  22
<212>  RNA
<213>  Homo Sapiens

<400>  8
ugagaacuga auuccauggg uu                                                22


<210>  9
<211>  22
<212>  RNA
<213>  Homo Sapiens

<400>  9
ccucugaaau ucaguucuuc ag                                                22


<210>  10
<211>  68
<212>  RNA
<213>  Homo Sapiens

<400>  10
gaggcaaagu ucugagacac uccgacucug aguaugauag aagucagugc acuacagaac        60

uuugucuc                                                                 68


<210>  11
<211>  22
<212>  RNA
<213>  Homo Sapiens

<400>  11
aaaguucuga gacacuccga cu                                                22


<210>  12
<211>  22
<212>  RNA
<213>  Homo Sapiens

<400>  12
ucagugcacu acagaacuuu gu                                                22

<210>  13
<211>  110
<212>  RNA
<213>  Homo Sapiens

<400>  13
ugaguuuuga gguugcuuca gugaacauuc aacgcugucg gugaguuugg aauuaaaauc        60

aaaaccaucg accguugauu guacccuaug gcuaaccauc aucuacucca                  110

<210>  14
<211>  110
<212>  RNA
<213>  Homo Sapiens

<400>  14
agaagggcua ucaggccagc cuucagagga cuccaaggaa cauucaacgc ugucggugag        60

uuugggauuu gaaaaaacca cugaccguug acuguaccuu gggguccuua                   110

<210>  15
<211>  23
<212>  RNA
<213>  Homo Sapiens

<400>  15
aacauucaac gcugucggug agu                                               23

<210>  16
<211>  22
<212>  RNA
<213>  Homo Sapiens

<400>  16
accacugacc guugacugua cc                                                22

<210>  17
<211>  110
<212>  RNA
<213>  Homo Sapiens

<400>  17
gccgagaccg agugcacagg gcucugaccu augaauugac agccagugcu cucgucuccc        60

cucuggcugc caauuccaua ggucacaggu auguucgccu caaugccagc                  110

<210>  18
<211>  21
<212>  RNA
<213>  Homo Sapiens

<400>  18
cugaccuaug aauugacagc c                                                 21

<210>  19
<211>  22
<212>  RNA
<213>  Homo Sapiens

<400>  19
cugccaauuc cauaggucac ag                                                22

<210>  20
<211>  71
<212>  RNA
<213>  Homo Sapiens

<400>  20
gcgacuguaa acauccucga cuggaagcug ugaagccaca gaugggcuuu cagucggaug        60

uuugcagcug c                                                            71

<210>  21
<211>  22
<212>  RNA
<213>  Homo Sapiens

<400>  21
uguaaacauc cucgacugga ag                                                22

<210>  22
<211>  22
<212>  RNA
<213>  Homo Sapiens

<400>  22
cuuucagucg gauguuugca gc                                                22

## Description

### FIELD OF THE INVENTION

The invention is based on the detection of microRNA (miR) in biological samples derived from subjects for assessing a risk that the subject develops a neurological and / or neuropsychological condition. The invention in particular detects the presence or absence and the amount of microRNA-181a-5p, microRNA146a-5p and/or microRNA148a-3p in such samples, which are shown by the invention to be associated with the neurological and / or neuropsychological condition. In addition, the invention provides a therapeutic application for the treatment of the neurological and / or neuropsychological condition by modulating the expression/function of these miR.

### DESCRIPTION

Impaired cognitive function is a key hallmark of age-associated neurodegenerative diseases and is often one of the first clinical symptoms. However, changes in cognitive function develop slowly over time and while some individuals develop pathological memory impairment others exhibit preserved cognitive function until old age, a phenomenon that has been referred to as cognitive reserve (Stern, 2012). As a result, pathological memory decline is often only diagnosed at an already advanced stage of molecular pathology. Bona fide examples are age-associated neurodegenerative diseases such as Alzheimer's disease (AD), the most common form of dementia in the elderly. The failure to detect risk individuals at an early stage of molecular pathology is considered to be a major reason why for example causative treatments for AD have so far failed in clinical trials (Abbott and Dolgin, 2016; Schneider et al., 2014). Thus, there is an urgent need for molecular biomarkers that could inform about cognitive status with the aim to detect individuals that are at risk for developing dementia to allow for earlier interventions.

In order to be applicable in the context of routine check-up screenings in a primary care setting such markers need to be comparatively inexpensive, easy to screen and predictive as to the identification of the individuals at risk. Such individuals could then be subjected to further diagnostics via more invasive and time-consuming examinations such as the analysis of cerebrospinal fluid (CSF) as well as functional and structural brain imaging (Molinuevo et al., 2018). Recent data suggest that biomarkers reflecting AD pathologies or neurodegeneration can be measured in blood (Olsson et al., 2016) (Blennow, 2017) (Jack et al., 2018) (Li and Mielke, 2019). However, age-associated cognitive diseases are multifactorial. Thus, there is additional need for molecular biomarker that could inform about the variable combinations of environmental and genetic factors that affect cognitive reserve and the progression to age-associated cognitive decline. A recent line of research indicates that circulating microRNAs might serve as diagnostic biomarker for various disorders (Witwer, 2015), including brain diseases (Rao et al., 2013) (Galimberti et al., 2014) (Hill and Lukiw, 2016; Kumar et al., 2017).

MicroRNAs that are 19-22 nucleotide long RNA molecules regulating protein homeostasis via binding to a target mRNA thereby causing its degradation or inhibition of translation (Gurtan and Sharp, 2013). MicroRNAs are particularly interesting as potential biomarker since microRNAome alterations reflect complex changes in cellular homeostasis and could therefore indicate the presence of multiple pathologies (Zampetaki et al., 2012) (Fischer, 2014a) (Condrat et al., 2020). Moreover, microRNAs are extremely stable in cell free environments, are resistant to thaw-freeze cycles (Mitchell et al., 2008) (Rao et al., 2013; Zampetaki et al., 2012) and have been implicated with learning and memory function, dementia and AD (Hebert et al., 2008) (Zovoilis et al., 2011) (Aksoy-Aksel A, 2014) (Jaber et al., 2019). In addition, RNA therapeutics is emerging as a promising approach to treat CNS diseases (Roovers et al., 2018) and there is evidence that microRNAs may be useful targets for RNA-based therapies (Zovoilis et al., 2011) (Banzhaf-Strathmann et al., 2014) (Salta and De Strooper, 2017) (Hanna et al., 2019).

Despite these promising data, the identification of a microRNA panel that could inform about cognitive status and help to detect patients at risk for developing cognitive impairment has been challenging.

Thus, it is an objection of the invention to develop a circulating microRNA signature that informs about differences in cognitive function and could help to identify patients at risk for developing dementia.

### BRIEF DESCRIPTION OF THE INVENTION

Generally, and by way of brief description, the main aspects of the present invention can be described as follows:

In **a first aspect,** the invention pertains a method for detecting or diagnosing a neurological and / or neuropsychological condition, or determining a risk of developing a neurological and / or neuropsychological condition, in a subject, the method comprising the steps of:
(i) Providing a biological sample of the subject;
(ii) Detecting at least the presence of at least one, preferably two, biomarker(s) selected from the group consisting of microRNA let-7b-5p, miR-130b-3p, miR-146a-5p, miR-148a-3p, miR-181a-5p, miR-192-5p and miR-30a-3p; preferably, in some particular embodiments, selected from the group consisting of microRNA-181a-5p, microRNA146a-5p and microRNA148a-3p, most preferably wherein the biomarker is a 3 microRNA panel consisting of microRNA-181a-5p, microRNA146a-5p and microRNA148a-3p; and optionally of at least one further biomarker selected from let-7b-5p, miR-130b-3p, miR-192-5p and miR-30a-3p, in the biological sample; and
(iii) wherein the detection of the biomarkers in the biological sample indicates the presence of the neurological and / or neuropsychological condition, or a risk of developing the neurological and / or neuropsychological condition, in the subject.

In **a second aspect,** the invention pertains to a microRNA (miR) antagonist composition for use in the prevention or treatment of a neurological and / or neuropsychological condition in a subject, wherein the microRNA antagonist composition comprises at least one miR antagonist of at least one microRNA selected from miR-146a-5p, miR-148a-3p, and miR-181a-5p.

In **a third aspect,** the invention pertains to a diagnostic kit for use in a method for detecting or diagnosing a neurological and / or neuropsychological condition, or determining a risk of developing a neurological and / or neuropsychological condition, in a subject, comprising primers or probes specific for the detection and/or quantification of the at least the biomarkers microRNA-181a-5p, microRNA146a-5p and microRNA148a-3p.

### DETAILED DESCRIPTION OF THE INVENTION

In the following, the elements of the invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains a method for detecting or diagnosing a neurological and / or neuropsychological condition, or determining a risk of developing a neurological and / or neuropsychological condition, in a subject, the method comprising the steps of:
(i) Providing a biological sample of the subject;
(ii) Detecting at least the presence of at least one, preferably two, biomarker selected from the group consisting of microRNA let-7b-5p, miR-130b-3p, miR-146a-5p, miR-148a-3p, miR-181a-5p, miR-192-5p and miR-30a-3p; preferably, in some particular embodiments selected from the group consisting of microRNA-181a-5p, microRNA146a-5p and microRNA148a-3p, most preferably wherein the biomarker is a 3 microRNA panel consisting of microRNA-181a-5p, microRNA146a-5p and microRNA148a-3p; and optionally of a further biomarker selected from let-7b-5p, miR-130b-3p, miR-192-5p and miR-30a-3p, in the biological sample; and
wherein the detection of the biomarkers in the biological sample indicates the presence of the neurological and / or neuropsychological condition, or a risk of developing the neurological and / or neuropsychological condition, in the subject.

MicroRNAs are short ribonucleic acids (RNA) found in eukaryotic cells. MicroRNAs regulate the expression of a gene in the body, are approximately 17 to 25 (preferably 19 to 22) nucleotides (hereinafter, "nt") in length, and are encoded by DNA. MicroRNAs may increase or reduce the expression of a specific protein after transcription from genomes and fragmentation. Mammalian microRNAs known so far may regulate insulin secretion, lymphocyte differentiation, cell division, cell death, viral replication and the like.

The term "microRNA" (or "miRNA", or "miR"), as used herein, has its art understood meaning as described before. MicroRNAs are a major group of noncoding RNAs that are known to regulate almost a third of all the coding genes. They are small endogenously formed repressors of gene expression. MicroRNAs usually bind to the 3' untranslated region (3'UTR) of the target RNA transcripts (mRNAs or circRNAs) and are capable of inducing posttranscriptional gene regulation by blocking translation or by degrading the target RNAs, or by doing both. MicroRNAs can also be chemically synthesized. In contrast to siRNA, which has perfect complementarity to the target RNA transcripts, miRNA binds imperfectly to the target RNA transcripts.

The most preferred biomarker of the present invention in all aspects and embodiments is a 3 microRNA panel consisting of microRNA-181a-5p, microRNA146a-5p and microRNA148a-3p.

In context of the present invention sequences of the microRNA biomarker of the invention are provided herein in SEQ ID NO: 1-22 according to the annotation provided herein below. Each microRNA has a matured sequence as well as one or more premature microRNA sequences.

According to one aspect of the present inventive concept, a method of diagnosing detecting or diagnosing a neurological and / or neuropsychological condition: providing a biological sample from a subject; measuring an amount of microRNA in the sample, the microRNA being at least one selected from the group consisting of microRNA-181a-5p, microRNA146a-5p and microRNA148a-3p; comparing the measured amount of the microRNA with a control (e.g., a measured amount of microRNA in a vesicle separated from a positive or negative control sample, or data representing the amount of microRNA in a positive or negative control sample); and providing a neurological and / or neuropsychological diagnosis based upon the comparison of the amount of the microRNA from the sample with the control.

Preferably the methods of the invention are not surgical methods, preferably are non-invasive methods, preferably performed ex-vivo or in-vitro.

The subject of the invention is preferably a mammal, preferably a human, more preferably wherein the subject has no immediate symptom, or mild symptoms, of the neurological and/or neuropsychological condition.

The biological sample may be, for example, urine, mucus, saliva, tears, blood plasma, blood serum, sputum, spinal fluid, lymph, tracheolar fluid, intestinal juice, genitourinary tract fluid, breast milk, semen, peritoneal fluid, cystic tumor fluid, amniotic fluid, or any combination thereof. The biological sample may contain a vesicle or be a vesicle itself. In preferred aspects or embodiments, the biological sample is selected from the group consisting of a fluid sample, preferably a fluid sample containing microRNA, such as a cerebrospinal sample, more preferably a blood sample, such as a whole blood, serum sample or plasma sample, or a fraction thereof such as a cell containing fraction or an extracellular vesicle (or exosomes) containing sample or isolated vesicles derived from such biological sample.

The biological sample may be obtained by blood collecting, sampling, biopsy, separating, or isolating the sample from the individual. The providing a biological sample may include separating or isolating vesicles from the obtained sample. The separating of the vesicle from the biological sample obtained may be performed, for example, using a solid support or a centrifugal force, a density gradient method, ultracentrifugation, filtration, dialysis, immunoaffinity chromatography using antibodies, free-flow electrophoresis, or a combination thereof. The separating of the vesicle from the biological sample may include incubating the vesicle together with, for example, a material specifically binding to the vesicle or a material that can intervene into the lipid bilayer of the vesicle. The incubating may be performed in vitro. In some embodiments, the separating of the vesicle from the biological sample may include washing.

In preferred embodiments step (ii) includes a step of quantifying the level of the biomarker, and wherein a differential level, preferably increased level, of the biomarker compared to a control or reference indicates the presence of the neurological and / or neuropsychological condition, or a risk of developing the neurological and / or neuropsychological condition, in the subject.

The amount of the microRNA may be measured using primers or a probe of a polynucleotide that is the same as or complementary to at least one microRNA (miRNA) selected from the group consisting of let-7b-5p, miR-130b-3p, miR-146a-5p, miR-148a-3p, miR-181a-5p, miR-192-5p and miR-30a-3p in a sample or a fragment of the microRNA, or of a preferred selection of microRNA biomarkers such as a 3 microRNA panel consisting of microRNA-181a-5p, microRNA146a-5p and microRNA148a-3p.The polynucleotide may be, for example, 2 nt to 25 nt, 3 nt to 24 nt, 4 nt to 23 nt, 5 nt to 22 nt, 6 nt to 21 nt, 7 nt to 20 nt, 8 nt to 19 nt, 9 nt to 18 nt, 10 nt to 17 nt, 11 nt to 16 nt, 12 nt to 15 nt, or 13 nt to 14 nt in length. The amount of the microRNA may be measured using, for example, a quantitative reverse transcription-polymerase chain reaction (qRT-PCR). Thus, in a preferred embodiment of the invention step (ii) is carried out by PCR, quantitative PCR (qPCR), RT-qPCR, droplet digital PCR, next generation sequencing or a hybridization based method such as Southern blot and/or lateral flow or microfluidic based assays.

The method of detecting or diagnosing a neurological and / or neuropsychological condition may include providing a such diagnosis based upon the comparison of the amount of the microRNA from the sample with a control. The providing a diagnosis may include determining whether the measured amount of the microRNA from the biological sample is greater or lesser than the amount of the microRNA from the control. For example, an increased amount of microRNA from the biological sample relative to a negative control sample is indicative of the presence or increased risk to develop a neurological and / or neuropsychological condition in the subject. The negative control may be an amount of microRNA from a biological sample from a subject having no such condition or a subject that is confirmed to not develop such condition. In preferred embodiments the control is an amount of microRNA from a biological sample from the subject obtained and measured at an earlier point in time. The increased amount of microRNA from the biological sample relative to the negative control (or the result of an earlier testing) may be an increase by more than about 0.2, 0.4, 0.5, 0.6, 0.8, 1.0, 1.2,1.4,1.6,1.8, 2.0, 2.2, 2.4, 2.6, or 2.8 Cp units.

In preferred embodiments the neurological and / or neuropsychological condition or neurodegenerative diseases according to the invention is associated with inflammatory processes and/or reduced neuronal plasticity. In some instances, the neurological and / or neuropsychological condition is associated with memory decline of the subject, preferably associated with age-associated memory decline, for example wherein the neurological and / or neuropsychological condition is a cognitive disorder, preferably selected from a form of dementia, such as Alzheimer's Disease (AD). Also, the neurological and / or neuropsychological condition may be associated with a reduced learning capability of the subject compared to a healthy subject.

In particular preferred embodiments of the invention pertain to a method of detecting or diagnosing a neurological and / or neuropsychological condition for determining a risk of the subject to develop the neurological and / or neuropsychological condition. The microRNA marker in accordance with the present invention are particular useful for monitoring a subject over a longer period of time (years) and to diagnose an immediate development of the neurological and / or neuropsychological condition in the subject, when the amount of selected microRNA is increases over time.

In **a second aspect,** the invention pertains to a microRNA (miR) antagonist composition for use in the prevention or treatment of a neurological and / or neuropsychological condition in a subject, wherein the microRNA antagonist composition comprises at least one miR antagonist of at least one miR selected from let-7b-5p, miR-130b-3p, miR-146a-5p, miR-148a-3p, miR-181a-5p, miR-192-5p and miR-30a-3p, preferably selected from miR-146a-5p, miR-148a-3p, and miR-181a-5p, and most preferably the miR is a 3 microRNA panel consisting of miR-146a-5p, miR-148a-3p, and miR-181a-5p.

The term "microRNA inhibitor" or "microRNA antagonist" refers to a molecule that prevents the binding between the microRNA and a RNA transcript target site. The present invention for in particular therapeutic purposes as described herein elsewhere relates to such microRNA inhibitors. In many preferred embodiments of the present invention, a microRNA inhibitor is a microRNA target site blocker. The term "microRNA target site blocker" refers to an antisense oligonucleotide that binds to the microRNA target site of a RNA transcript (mRNA or circRNA) thereby preventing microRNAs from gaining access to that site. MicroRNA target site blockers allow researchers to study the effects of a microRNA on a single target. In contrast, the phenotype observed with inhibiting a microRNA reflects the combined effects of that microRNA on all its targets. MicroRNA target site blockers may also be termed MRE inhibitors. The term "microRNA recognition element or MRE" refers to the cis-sequence element present on the RNA transcript (mRNA or circRNA) that is recognized by miRNA, mediating their binding. The term "MRE inhibitor" refers to a short-modified RNA molecule, that is resistant to RNAse H and that is capable of preventing binding of miRNA to its target by interacting directly to the MRE with high affinity. Any such compounds shall form part of the present invention.

In certain embodiments, the miR antagonist is a oligo-nucleic acid agent capable of reducing microRNA levels of a microRNA selected from the group consisting of let-7b-5p, miR-130b-3p, miR-146a-5p, miR-148a-3p, miR-181a-5p, miR-192-5p and/or miR-30a-3p, preferably selected from miR-146a-5p, miR-148a-3p, and miR-181a-5p in a cell by an amount (expressed in percentage) of at least 10-20%, at least 30-40%, at least 50-60%, at least 70-80%, at least 90-98%, or at least 99% when the oligonucleic acid agent is introduced into a mammalian cell.

In certain embodiments of the invention a microRNA inhibitor or antagonist of the invention is a molecule, such as a nucleic acid containing molecule, that hybridizes under stringent conditions to, and thereby inhibits or antagonizes, a microRNA sequence shown in any one of SEQ ID NO: 1-22, or alternatively that hybridizes under stringent conditions to a target mRNA of such microRNA, and thereby inhibits or antagonizes the function of the corresponding microRNA.

The oligo nucleic acid agents of the invention are defined by their sequence; however, the skilled person understands that by exchanging one or two positions, particularly while increasing binding to the mRNA through introduction of nucleotide analogues, sufficient specificity of binding may be attained to achieve the inventive effect.

In certain embodiments, the oligonucleic acid agent comprises a sequence hybridizing to a microRNA selected from the group consisting of let-7b-5p, miR-130b-3p, miR-146a-5p, miR-148a-3p, miR-181a-5p, miR-192-5p and/or miR-30a-3p, preferably selected from miR-146a-5p, miR-148a-3p, and miR-181a-5p. The agent sequence is at least 95% identical, particularly 96%, 97%, 98%, 99% or 100% identical to SEQ ID 001. In certain embodiments, the hybridizing sequence comprises deoxynucleotides, phosphothioate deoxynucleotides, LNA and/or PNA nucleotides or mixtures thereof.

Antisense composed partially or entirely of nucleoside analogues

In certain embodiments, the oligonucleic acid agent is an antisense oligonucleotide.

In certain embodiments, the oligonucleic acid agent comprises or is essentially composed of LNA moieties and comprises about 20 or fewer nucleotides.

In certain embodiments, the oligonucleic acid agent for use in a method of treatment or prevention of a condition mentioned herein, or essentially consists of one or several peptide nucleic acid (PNA) moieties. In certain embodiments, the oligonucleic acid agent of the invention may include a phosphonate, a phosphorothioate or a phosphate ester phosphate backbone modification. In certain embodiments, the oligonucleic acid agent of the invention may include ribonucleotides. Optionally, the oligonucleic acid agent of the invention may include deoxy ribonucleotides. In certain embodiments, the hybridizing sequence of the oligonucleic acid agent comprises ribonucleotides, deoxynucleotides, phosphothioate deoxynucleotides, phosphothioate ribonucleotides and/or 2'-o-methyl-modified phosphothioate ribonucleotides.

In certain embodiments, the oligonucleic acid agent comprises ribonucleotides and deoxyribonucleotides, in particular modified ribonucleotides and modified deoxyribonucleotides. A non-limiting example of a modification of deoxyribonucleotides and ribonucleotides are phosphorothioate modified linkages in the oligonucleotide backbone. A non-limiting example of a modification of ribonucleotides is a 2'-o to 4'-C bridge.

In certain embodiment the 2'-o/4'-C bridge is a five-membered, six-membered or seven membered bridged structure.

In certain embodiments, the oligonucleic acid agent is a gapmer characterized by a central DNA block, the sequence of which is complementary to the microRNA of the invention, and which is flanked on either side (5' and 3') by nuclease-resistant LNA sequences which are also complementary to the microRNA. The central DNA block contains the RNase H activating domain, in other words is the part that lead the target DNA to be hydrolyzed. In certain embodiments, the flanking LNA is fully phosphorothioated.

In certain embodiments, the oligonucleic acid agent comprises 12 - 20 nucleotides. In certain particular embodiments, the oligonucleic acid agent comprises 14-16 nucleotides. In certain embodiments, the hybridizing sequence of the oligonucleic acid agent according to the invention comprises 14,15 or 16 nucleotides.

In certain embodiments, the central deoxyribonucleotide oligomer block of the gapmer comprises at least 5 deoxyribonucleotides. In certain embodiments, the central deoxyribonucleotide oligomer block of the gapmer comprises 5 to 10 deoxyribonucleosides linked by phosphate ester bonds or thiophosphate ester bonds. In certain embodiments, the central deoxyribonucleotide oligomer block of the gapmer comprises a phosphate backbone between the deoxyribonucleosides. In certain embodiments, the oligonucleic acid agent comprises, or essentially consists of, a central block of 5 to 10 deoxyribonucleotides linked by phosphate ester bonds flanked on either side by 2'-o modified ribonucleotides or PNA oligomers. In certain embodiments, the oligonucleic acid agent comprises, or essentially consists of, a central block of 5 to 10 deoxyribonucleosides flanked by LNA nucleoside analogues. In certain particular embodiments, said LNA nucleoside analogues are linked by phosphothioate moieties.

In certain embodiments, the oligonucleic acid agent is a ribonucleic agent, particularly a siRNA or shRNA. An RNA interference (RNAi) agent in the context of the present specification refers to a ribonucleotide oligomer that causes the degradation of its enhancer RNA (eRNA) target sequence. In certain embodiments, the RNAi agents of the invention comprise, or consist of,
- a single-stranded or double-stranded interfering ribonucleic acid oligomer or precursor thereof, comprising a sequence tract complementary to the targeted enhancer RNA molecule; or
- a single-stranded or double-stranded antisense ribonucleic or deoxyribonucleic acid, comprising a sequence tract complementary to the targeted enhancer RNA molecule. In certain embodiments, the sequence tract complementary to the targeted enhancer RNA molecule is a contiguous sequence tract 7, 8, 9, 10, 11, 12, 13, 14, 15, 16, 17, 18, 19, 20, 21 or 22 nucleotides in length.

In certain embodiments, the RNAi agents of the invention include, but are not limited to, small interfering RNAs (siRNAs), short hairpin RNAs (shRNAs), microRNAs and non-coding RNAs or the like, Morpholinos (phosphodiamidate morpholino oligomers) and Dicer substrate siRNAs (DsiRNAs, DsiRNAs are cleaved by the RNAse III class endoribonuclease Dicer into 21 - 23 base duplexes having 2-base 3'-overhangs), UsiRNAs (UsiRNAs are duplex siRNAs that are modified with non-nucleotide acyclic monomers, termed unlocked nucleobase analogues (UNA), where the bond between two adjacent carbon atoms of ribose is removed), self-delivering RNAs (sdRNAs) including rxRNA^{™} (RXi Pharmaceuticals, Westborough, MA, USA).

In some embodiments, the RNAi agents of the invention comprise analogues of nucleic acids such as phosphotioates, 2'O-methylphosphothioates, peptide nucleic acids (PNA; N-(2-aminoethyl)-glycine units linked by peptide linkage, with the nucleobase attached to the alpha-carbon of the glycine) or locked nucleic acids (LNA; 2'O, 4'C methylene bridged RNA building blocks). The hybridizing sequence may be composed partially of any of the above nucleotides, with the rest of the nucleotides being" native" ribonucleotides occurring in nature, or may be mixtures of different analogues, or may be entirely composed of one kind of analogue. In certain embodiments, the oligonucleic agent is conjugated to, or encapsulated by, a nanoparticle, a virus and a lipid complex. In certain embodiments, the oligonucleic acid agent is a gapmer comprising a central deoxyribonucleotide oligomer block flanked by nuclease resistant ribonucleotide analogues on either (5' and 3') side. Within the scope of the present invention is a method for treating or preventing heart disease in a patient in need thereof, comprising administering to the patient an oligonucleic acid agent according to the invention.

Dosage forms may be for enteral administration, such as nasal, buccal, rectal, transdermal or oral administration, or as an inhalation form or suppository. Alternatively, parenteral administration may be used, such as subcutaneous, intravenous, intrahepatic or intramuscular injection forms. Optionally, a pharmaceutically acceptable carrier and/or excipient may be present. In certain embodiments, the amount of the oligonucleic acid agent sufficient to reduce expression of one or more microRNAs is of 1 nanomolar or less, 200 picomolar or less, 100 picomolar or less, 50 picomolar or less, 20 picomolar or less, 10 picomolar or less, 5 picomolar or less, 2, picomolar or less and 1 picomolar or less in the environment of the cell.

The miR antagonist composition of the invention may comprises miR antagonists, either RNAi or an oligonucleic acid agent, against (or targeting, or hybridizing to) microRNA-181a-5p, microRNA146a-5p and microRNA148a-3p.

A subject for therapeutic treatment of the invention is a mammal, preferably a human, more preferably wherein the subject has no immediate symptom, or mild symptoms, of the neurological and / or neuropsychological condition, but which was diagnosed of having a risk of developing the neurological and / or neuropsychological condition, or which already developed mild or severe symptoms of the neurological and / or neuropsychological condition.

In **a third aspect,** the invention pertains to a diagnostic kit for use in a method for detecting or diagnosing a neurological and / or neuropsychological condition, or determining a risk of developing a neurological and / or neuropsychological condition, in a subject, comprising primers or probes specific for the detection and/or quantification of the at least the biomarkers microRNA-181a-5p, microRNA146a-5p and microRNA148a-3p.

According to an aspect of the present inventive concept, a composition or kit for detecting or diagnosing a neurological and / or neuropsychological condition comprises at least one polynucleotide having a sequence that is the same as, or complementary to, a microRNA (microRNA) selected from the group consisting of let-7b-5p, miR-130b-3p, miR-146a-5p, miR-148a-3p, miR-181a-5p, miR-192-5p and miR-30a-3p, preferably selected from miR-146a-5p, miR-148a-3p, and miR-181a-5p, and most preferably the microRNA is a 3 microRNA panel consisting of miR-146a-5p, miR-148a-3p, and miR-181a-5p, in a biological sample, or a fragment of the microRNA. The composition can comprise two or more, three or more, four or more, five or more, six or more, seven or more, eight or more, nine or more, ten or more, or 20 or more vesicles each containing a different polynucleotide having a sequence that is the same as or complementary to a microRNA (miRNA) selected from the group consisting let-7b-5p, miR-130b-3p, miR-146a-5p, miR-148a-3p, miR-181a-5p, miR-192-5p and miR-30a-3p, preferably selected from miR-146a-5p, miR-148a-3p, and miR-181a-5p, and most preferably the microRNA is a 3 microRNA panel consisting of miR-146a-5p, miR-148a-3p, and miR-181a-5p, or a fragment of the microRNA. Each possible combination of two or more of these miRNA, their fragments and/or sequences complementary thereto is disclosed herewith, even though not explicitly mentioned. The composition can comprise other additional components, such as other vesicles containing different nucleic acids or other materials.

Preferably, all herein mentioned microRNAs are human microRNAs.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

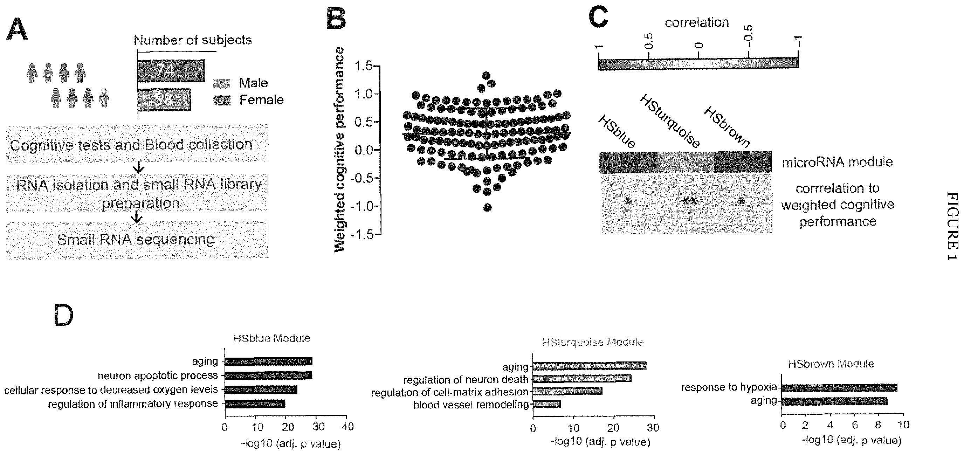

**Figure 1****: Circulating microRNA expression levels correlate with cognitive function in healthy humans. A**. Experimental approach for the detection of blood microRNAs that correlate with memory function in healthy humans. This cohort includes 132 healthy and young individuals (74 male and 58 females, age: 25.95 ± 5.1 years). Participants took part in cognitive tests and donated blood samples (PAXgene tubes). **B**. Weighted cognitive score of the 132 individuals shows the expected variability. **C**. Co-expression analysis revealed 3 microRNA clusters that were significantly linked to weighted cognitive performance. Pearson Correlation. **D**. The analysis of the experimentally validated mRNA targets of the microRNAs belonging to 3 clusters identified in (C). Downstream analyses on those genes reveal that they control processes related to aging and age-related functions known to play a role in cognition. **P* < 0.05; ***P* < 0.01.

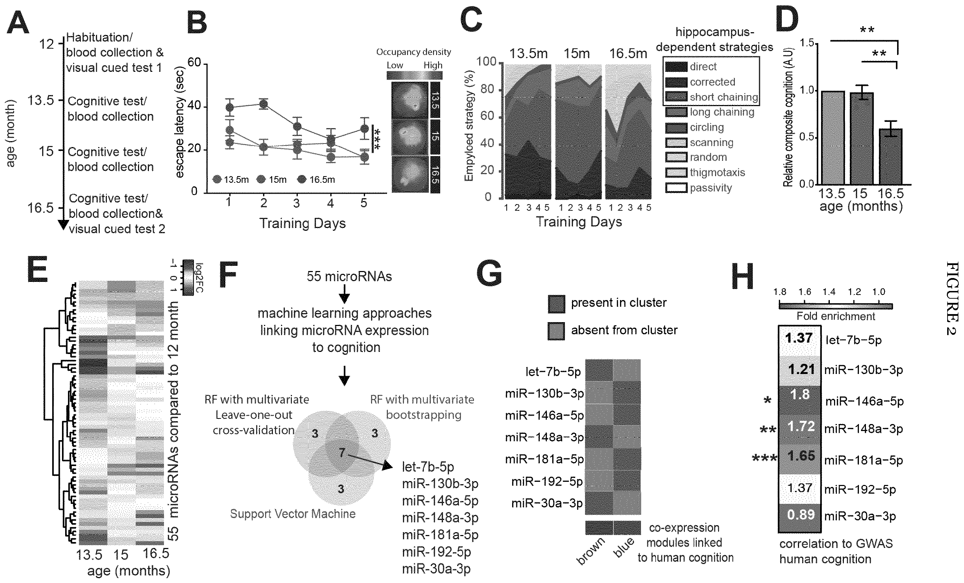

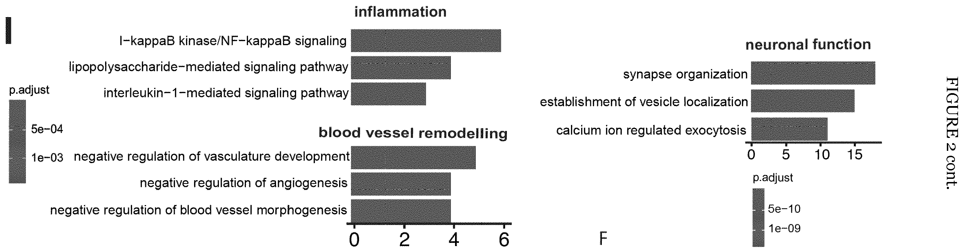

**Figure 2****: Identification of circulating microRNAs informative about memory decline in mice. A**. Experimental design of the water maze experiment. At 12 months of age, mice were subjected to the water-maze training protocol in order to habituate the animals to the procedure. Subsequently, mice were subjected to water maze training followed by a probe test at 13.5, 15 and 16.5 months of age. The platform position was altered during each training procedure. Blood was collected upon completion of each water maze procedure. A visual cued test was performed after the first and after the last blood collection when mice were 12 and 16.5 months of age respectively. **B**. Escape latency during water maze training when mice were 13.5, 15 or 16.5 months of age (n = 10 each group). Two-way ANOVA followed by Tukey's multiple comparisons test revealed significant effects of training trials (p value < 0.0001) and age (p value 0.0004) on the performance. On day one and two, there was a significant (p value < 0.05) difference between mice at 13.5 vs. 16.5 months and 15 vs. 16.5 months of age (n = 10 each group). On the 5^{th} day of training there was a significant difference observed between 15 and 16.5 months of age. Density plot (Right) shows the occupancy pattern of mice at different time points of aging. Occupancy signal on the platform was the least at 16.5 months, suggesting mice at the given age failed to locate the platform. **C**. Analysis of the different search strategies during the water training sessions. Note that especially at 16.5 months of age mice adopt hippocampal independent search strategies indicative of impaired cognitive function. **D**. The cumulative cognitive score calculated for each day on the basis of hippocampal dependent strategies was significantly impaired when comparing mice at 16.5 month of age to their performance at 15 or 13.5 months of age. Data is normalized to 13.5 months group (Ordinary One-way ANOVA, Tukey's multiple comparison test). **E**. Heat map showing the expression pattern of 55 aging responsive microRNAs significantly deregulated during the course of aging. All data is shown in comparison to the expression level at 12 month of age **F**. Expressions of these 55 microRNAs and cognitive performances along aging were used to identify microRNA features linked to cognition. Three independent approaches [e.g. random forests (RF) with leave one out cross validation, RF with multivariate bootstrapping and support vector machine (SVM)] find seven common microRNAs that explain cognitive variability assayed in the water maze test. **G**. Color map showing that the 7 microRNAs identified in (F) are present in the co-expression modules significantly linked to cognition in healthy humans as described in Fig 1. **H**. Heat map showing the enrichment of mRNA targets of the 7 microRNAs shown in (G) with a gene-set identified by GWAS studies linking genes to cognition in healthy humans (Davies G, 2018). Note that target genes of microRNA-181a-5p, microRNA-148a-3p and microRNA-146a-5p are significantly overlapped in a hypergeometric test (Fold enrichment > 1.5 and FDR<0.05). **I**. Gene ontology analyses of microRNA-181a-5p, microRNA-148a-3p and microRNA-146a-5p targets reveal processes linked to inflammation, vascular remodeling and neuronal function. Error bars indicate mean ± SEM.

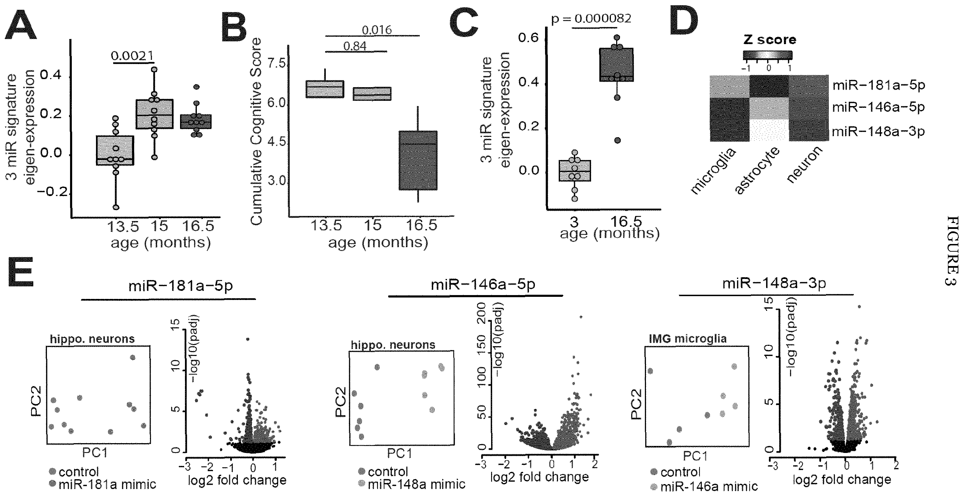

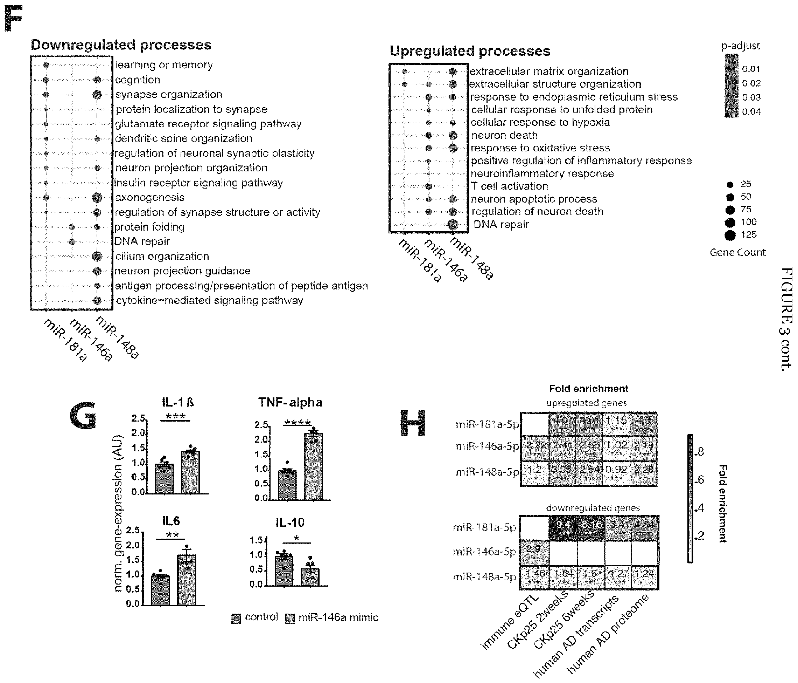

**Figure 3****. Expression changes of 3-miR signature reflect aberrant neuronal and immune processes. A**. Eigen value of the 3-microRNA signature measured in the mice that performed longitudinal water maze training. Note the significantly increased expression of the signature already at 15 months of age, suggesting that increased expression levels precede detectable cognitive impairment. **B.** Cognitive score measured in the same mice reveals cognitive decline between 15 and 16,5 months of age (p value 0.0079). **C**. Eigen value showing the expression of the 3-microRNA signature in the hippocampus of 3 and cognitively impaired 16,5 months old mice. **D.** Relative enrichment of the three microRNAs in microglia, astrocytes and neurons based on the data from a previous study. **E**. Overexpression of microRNAs in relevant cell types for 48 hours followed by genome wide RNA-seq analysis. micro-181a-5p was overexpressed in primary hippocampal neurons, while micro-146a-5p was overexpressed in microglia culture. Immortalized microglial cell line was used for this purpose. Given that microRNA-148a-3p was highly enriched in neurons (D), primary hippocampal neurons were treated with the corresponding microRNA-148a-3p mimic. PCA plot shows that the mimic and control treated samples cluster distinguishingly separate from one another. Volcano plot displays the genes significantly deregulated in mimic treated samples compared to control samples (FDR <0.05). Red color indicates the up-regulated genes while the blue color represents the genes those were down-regulated. **F**. Gene ontology analyses for up- and down-regulated genes. Over-expression of microRNA-181a-5p and microRNA-148a-3p led to down-regulation of genes related to cognition and synaptic functions, while downregulated genes due to increased expression of microRNA-146a-5p represent DNA repair and protein folding mechanisms. The up-regulated genes due to overexpression of these microRNAs represent several processes including extracellular matrix, stress responses and neuronal death. Interestingly, microRNA-146a-5p overexpression in microglia led to increased expression of inflammatory related genes. Size of the dot represents the number of genes belonging to the given process and the color represent the p value after multiple corrections. **G**. qPCR analysis confirms the over-expression of pro-inflammation related genes (IL-1β, IL-6, TNF-alpha) due to overexpression of microRNA-146a-5p. Expression of anti-inflammatory gene, IL-10 was downregulated in mimic treated cells compared to the controls. Unpaired t-tests, two-tailed, ****P<0.0001, ***P<0.001, **P<0.01, *P<0.05 **H.** Hypergeometric overlap of the up- and down-regulated genes (E) with gene-sets from different datasets. The inventors calculated enrichment of the deregulated genes relative to those gene-sets and used a Fisher's exact test p value after multiple adjustments to estimate the significance of the overlap. Immune related genes based on expression quantitative loci (e-QTL) were retrieved from a previous study. RNAseq data from CKp25 mice at 2 and 6 weeks after induction were retrieved from GSE65159 and up- and downregulated genes compared to littermate controls were determined after differential expression (significant genes; adjusted p value<0.05). Up- and downregulated transcripts in human AD patients compared to control subjects were determined by analysis of the available data (GSE44770). Proteins those are over- and reduced-expressed in AD patients compared to controls were retrieved from a previous study. Overlap analysis for the up- and down-regulated genes due to over-expression of microRNAs were performed to those separately from the disease conditions. Human orthologs of the mouse deregulated genes were used to perform the overlap analysis in human datasets. Color code represents fold enrichment. *FDR<0.05, **FDR<0.01, ***FDR<0.001

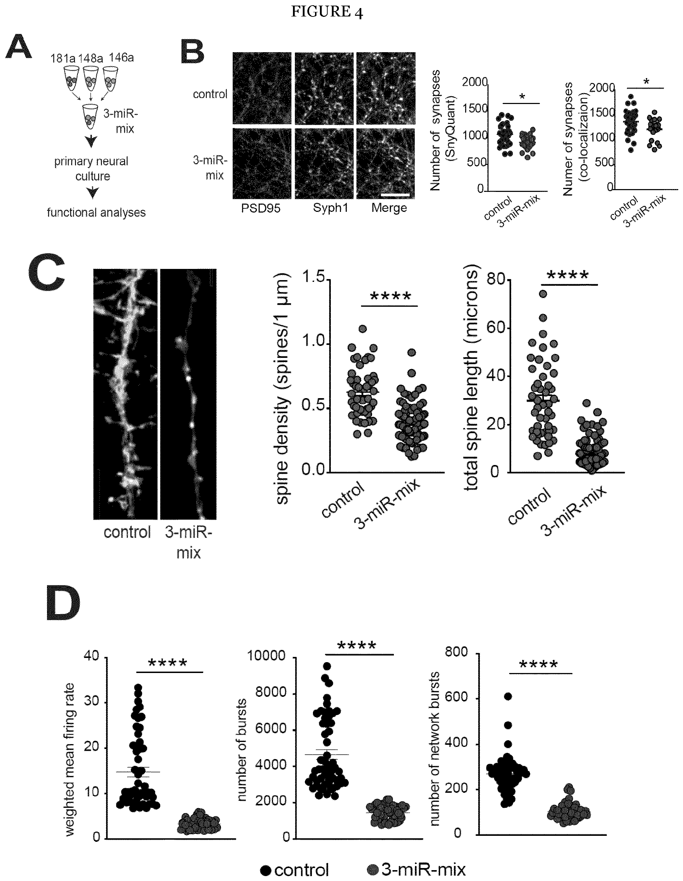

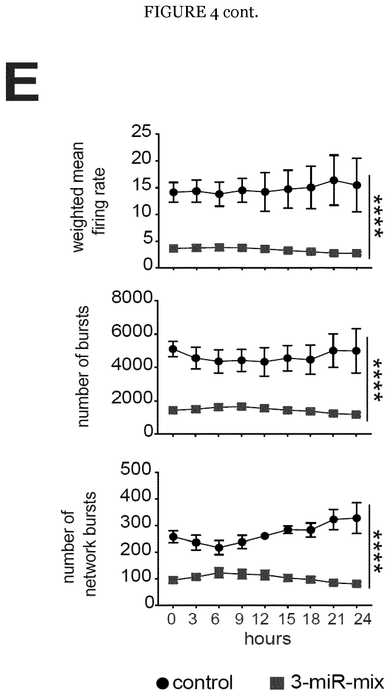

**Figure 4****: Increased expression of 3-microRNA signature perturbs synaptic organization and neuronal activity. A**. Primary hippocampal neurons were treated with a mixture of 3-miR mimic or control oligonucleotides and follow up analyses (imaging, electrical recordings) were performed. **B**. Functional mature synapses were quantified via co-localizations of pre- (Synatophysin 1) and the post-synaptic (PSD-95) markers and compared between 3-miR-mix and control groups. Scale bar: 10 µm. Two independent methods (SynQuant and Colocalization) were used for quantification. 3-miR-mix reduced the number of functional synapses compared to controls (n = 24-30 images) **C**. Dendrite labeling and quantification. Dendritic spines were stained with Dil. Scale bar: 10 µm. Spike density and total spine length are substantially reduced in 3-miR mix treated primary neurons compared to those treated with scramble RNA (n = 49-97 images) **D**. Hippocampal neurons were cultured in a multi electrode array (MEA) plate equipped with sixteen electrodes. Spontaneous activity of the neurons was recorded at every 3 hours (10 minutes/session) for 24 hours. Weighted mean firing rate, number of bursts, and network bursts are significantly decreased in neurons treated with 3-miR-mix compared to control. **E**. The aberrant neuronal firing activity (weighted mean firing rate) and reduced number of bursts and network bursts were observed across the 24 hour of time period. Unpaired t-tests, two-tailed. Error bars indicate mean ± sem. *P<0.05, ****P<0.0001

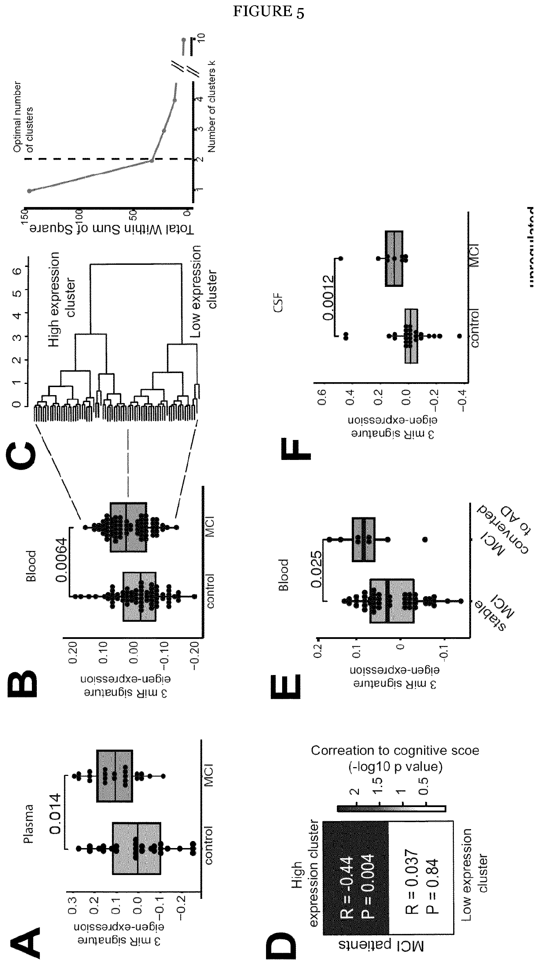

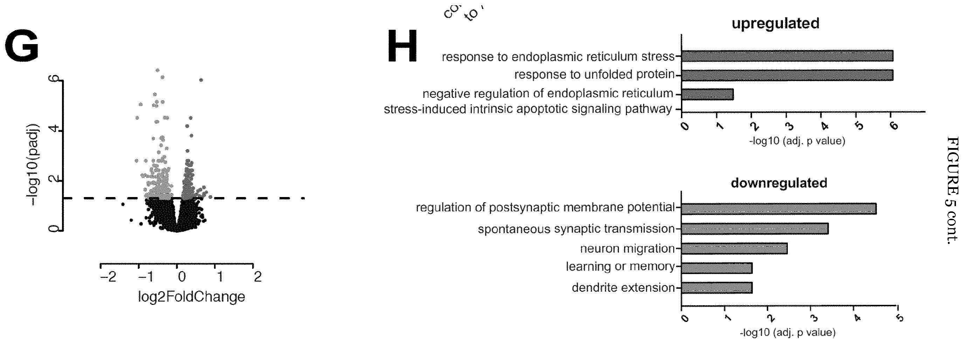

**Figure 5****: Expression of 3-microRNA signature in human patients. A**. Eigen value showing the increased expression of the 3-microRNA signature in blood plasma samples of age matched MCI (n = 23) patients compared to controls (n = 27). **B**. Expression of 3-microRNA signature is increased in PAXgene blood samples of MCI patients (n=73) compared to controls (n=67) from the DELCODE cohort. Effect of age, gender, sequencing bias, and other latent covariates were regressed prior to eigenvalue construction. **C**. Clustering of eigen-expression identifies two expression clusters in MCI patients based on elbow method of detecting optimum number of clusters. **D**. Patients representing the cluster with higher expression of 3-miR eigenvalue shows negative correlation with their weighted cognitive score (cor = 0.444, p = 0.004). In contrast, patients with low expression of 3-miR signature did not show significant correlation with the cognitive score (p=0.84). **E**. Eigen value showing the expression the 3-microRNA signature in MCI patients for those the follow up diagnostics data assessed 2 years after were available. 14% of these MCI patients developed Alzheimer's disease (AD), while the rest 86% patients remained with MCI (stable MCI). The boxplot depicts the increased expression levels of 3-miR signature in patients who converted to AD (n=8) compared to those that had stable MCI (n=47). **F**. Increased expression of 3-microRNA signature in cerebrospinal fluid (CSF) of MCI patients (n = 9) compared to controls (n = 26). Sequencing data were adjusted for the effects due to age, gender, sequencing batches and latent covariates as determined by sva before eigenvalue calculation. Wilcoxon-rank test, P value is given on the corresponding panel. **G**. Human bioengineered neuronal organoids (BENOs) were treated at DIV 60 with the 3-miR-mix or corresponding controls for 24 hours and RNAseq was performed from prepared RNA. Volcano plot displays the significant deregulated genes in BENOs after over-expressing the 3-miR-mix (FDR<0.05). **H**. Gene ontology shows significant up- and downregulated processes based on the differentially expressed genes. X-axis represents the - log₁₀ of adjusted p value.

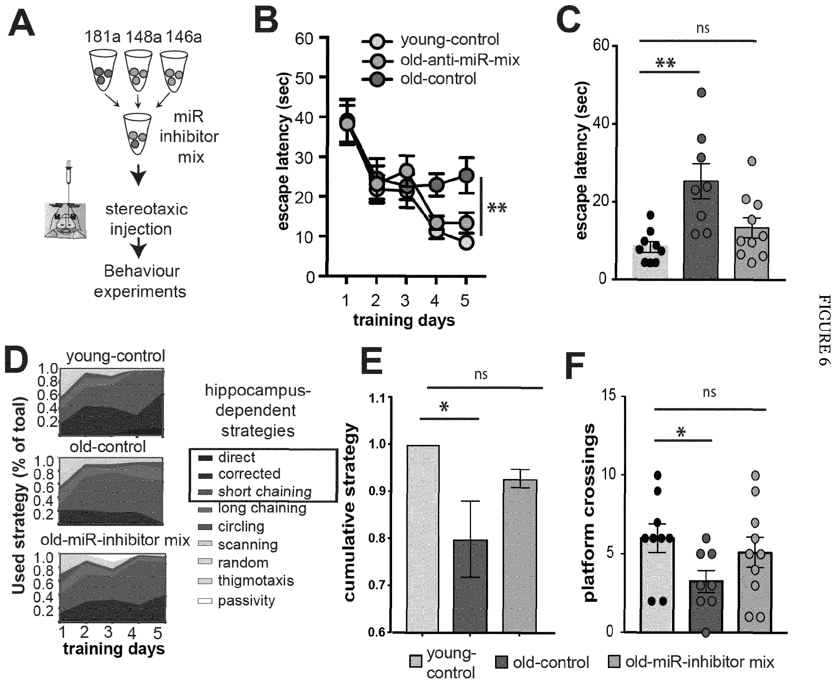

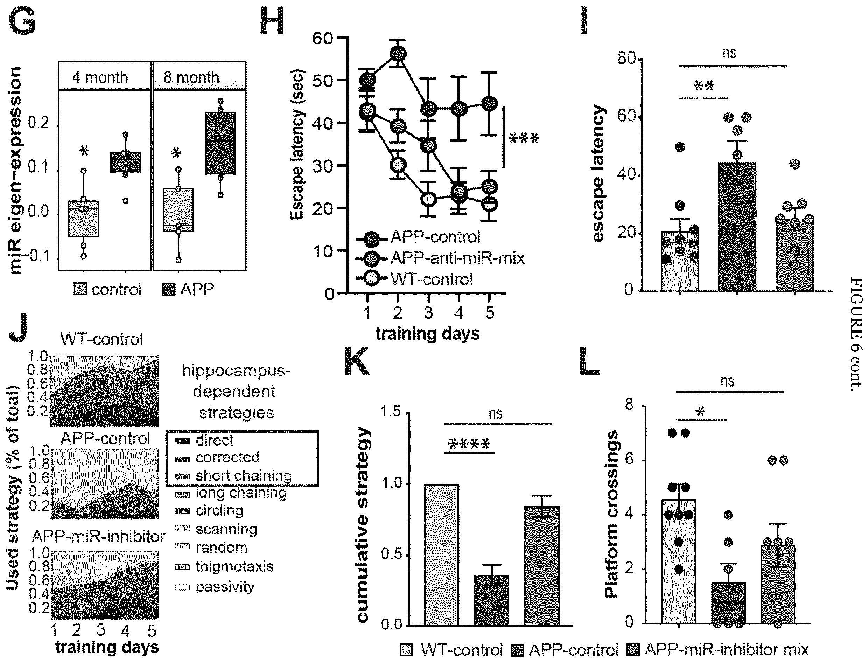

**Figure 6****: Targeting the 3-microRNA signature reinstates cognitive function in mouse models for dementia. A**. Experimental design. Inhibitor mixture for 3-microRNAs (anti-miR mix) namely microRNA-181a-5p, microRNA-148-3p and microRNA-146a-5p was injected into the dorsal hippocampus prior to behavioral testing. As control, scramble siRNAs were injected as described above. Experiments were performed in two mouse models representing Aging and Alzheimer's disease (AD). B. Escape latency during the water maze training comparing 3-month old (young-control) mice and 16,5-month old (old-control) injected with scramble control oligonucleotides and 16,5-month old mice injected with microRNA inhibitors (old miR-inhibitor mix). C. Escape latency on final day of water maze training was impaired improved in old mice treated with the microRNA inhibitors mix. **D**. Depiction of the search strategies during the water maze training. **E**. The cumulative cognitive score calculated for each day on the basis of hippocampal dependent strategies was significantly impaired when comparing mice old control mice to young control mice. Data is normalized to young control group **F**. Number of visits to the platform during the probe test **G**. Eigen value showing the expression of the 3-microRNA signature in the hippocampus of 4 and 8 months old APPPS1-21 mice. **H**. Escape latency during the water maze training comparing 7 months old wild type mice (WT-control) and APPPS-21 mice (APP-control) injected with scramble control oligonucleotides and APPPS1-21 mice injected with microRNA inhibitors (APP miR-inhibitor mix). **I**. Escape latency measured on the last day of water maze training was reduced in APP-control mice. However, learning performance was rescued in APP miR-inhibitor mix mice. **J.** Depiction of the search strategies during the water maze training in experimental groups. **K.** The cumulative cognitive score calculated for each day on the basis of hippocampal dependent strategies was significantly impaired when comparing WT control mice to APP control mice. Data is normalized to WT control group. **L**. Comparison of the number of visits to the platform during probe test. Error bars indicate mean ± SEM. (B, H) Two-way ANOVA, (C, E, I, K, L) One-way ANOVA, unpaired t-tests, two tailed (F-G). *P<0.05, **P<0.01, ***P<0.001, P<0.0001, n = 6-10.

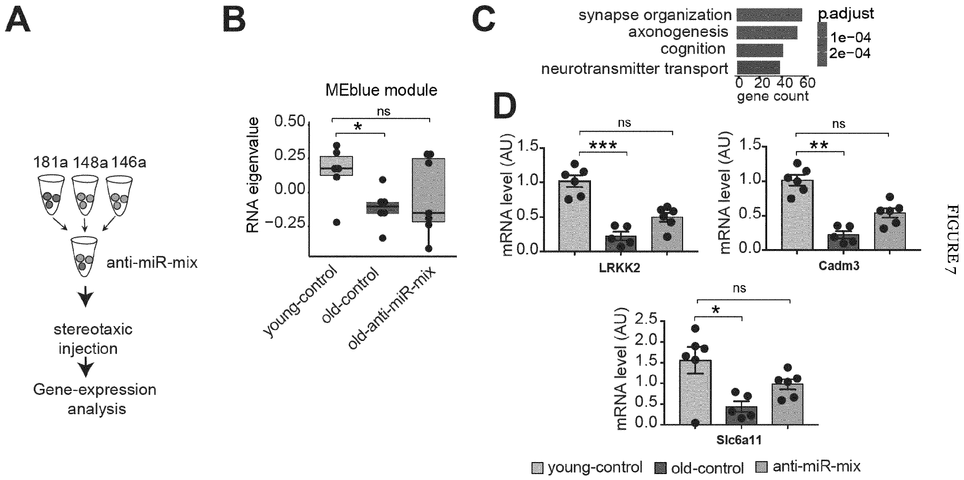

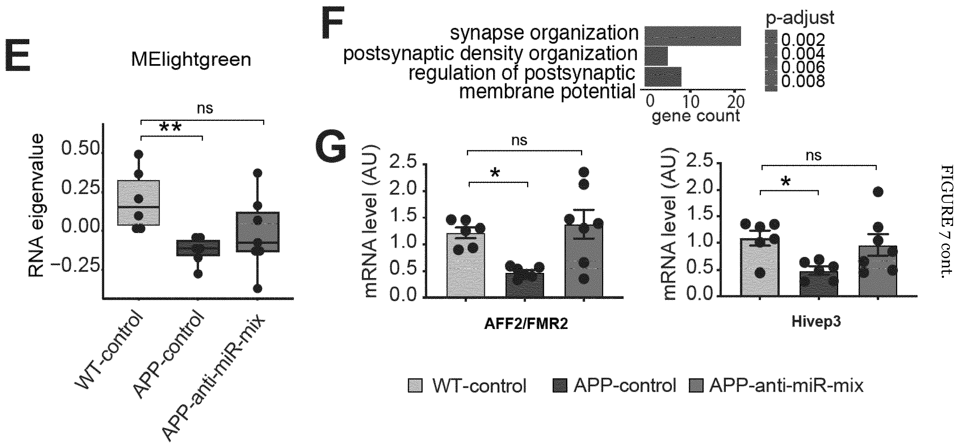

**Figure 7****: The 3-microRNA signature is a target for RNA therapeutics to treat neuronal dysfunction. A.** Experimental outline. Anti-miR-mix of the 3 microRNAs was injected into the dorsal hippocampus of the mice as previously described. RNAseq data were generated from dorsal hippocampal tissues and compared to those treated with control scramble oligonucleotides. **B.** Weighted gene co-expression analysis of hippocampal RNA-seq data identified the MEblue gene cluster that is decreased when comparing 3-months old mice (young-control) to cognitively impaired 16,5-months old mice (old -control) with a scramble control oligonucleotide injected. Treating old mice with the miR-inhibitor mix (old miR-inhibitor mix) reinstated gene-expression of this cluster, at least in part (n = 6-7, Kruskal-wallis test). **C**. Gene ontology reveals that the MEblue cluster is linked to cognition and synapse organization. **D.** qPCR assay for several synaptic genes (LRKK2, Cadm3 and Slc6a11) confirms reinstatement of gene expression with anti-miR-mix (n = 5-7, Kruskal-wallis test). **E.** Weighted gene co-expression analysis of hippocampal RNA-seq data identified a MElightgreen gene cluster that is decreased when comparing wilt type control (WT control) to APPPS1-21 mice (APP-control) and was reinstated in APPPS1-21 mice treated miR-inhibitor mix (n = 6-7, Kruskal-wallis test). **F.** Gene ontology reveals that the MElightgreen cluster is linked to cognition and synapse organization. **G.** qPCR data shows rescue of AFF2/FMR2 and Hivep3 expression in APP/PS1 mice treated with inhibitor cocktail (n = 6-7, Kruskal-wallis test). *P<0.05, **P<0.01, ***P<0.001. Error bars indicate mean ± SEM.

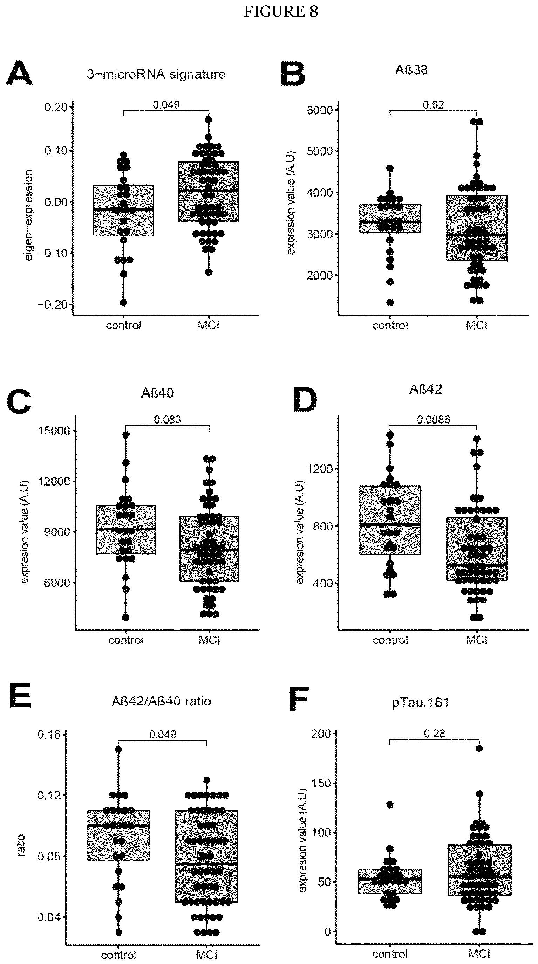

**Figure 8****: Comparison of the 3-microRNA signature in blood with established CSF AD biomarker within the same control and MCI patients.** Please note that expression data of the CSF-biomarker were not available for all the samples used in Fig 5B. Therefore, the comparative analysis of the blood 3 microRNA signature and the CSF biomarker was performed on control and MCI patients for which all datasets were available; control = 24, MCI = 52. A. Eigenvalue of 3-microRNA signature in blood is significantly increased in MCI patients compared to controls. Levels of (B) Aβ38 and (C) Aβ40 did not change significantly between patients and control subjects. Both (D) Aβ42 alone and (E) Aβ42/40 ratio were significantly different between control and MCI patients. F. There was no statistically significant difference in the levels of phosphorylated Tau 181 when comparing MCI patient vs controls. Since Tau could be considered a marker for neurodegeneration, it is not unexpected that changes in Aβ precede changes in Tau pathology. Unpaired t-tests, two-tailed. These data suggest that the 3-microRNA signature is comparable to more invasive established AD biomarker.

The sequences show:
The sequences show the mature microRNA sequences of the microRNA biomarker of the invention, and their precursor microRNA sequences. Precursor sequences are indicated by the denomination "_pre". MicroRNA sequences and additional details can be derived from "www.mirbase.org", the microRNA database (Ana Kozomara and Sam Griffiths-Jones; miRBase: integrating microRNA annotation and deep-sequencing data. Nucleic Acids Res. (2010); in the database version derived from the website on December 28, 2020). MiR-base accession numbers (MI... or MIMAT...) are indicated below.
**SEQ ID NO: 1** Human let 7b hsa-let-7b_pre MI0000063 
CGGGGUGAGGUAGUAGGUUGUGUGGUUUCAGGGCAGUGAUGUUGCCCCUCGGAAGAUAA
CUAUACAACCUACUGCCUUCCCUG
**SEQ ID NO: 2** hsa-let-7b-5p MIMAT0000063
   UGAGGUAGUAGGUUGUGUGGUU
**SEQ ID NO: 3** hsa-let-7b-3p MIMAT0004482
   CUAUACAACCUACUGCCUUCCC
**SEQ ID NO: 4** hsa-mir-130b_pre MI0000748 
GGCCUGCCCGACACUCUUUCCCUGUUGCACUACUAUAGGCCGCUGGGAAGCAGUGCAAUGA
UGAAAGGGCAUCGGUCAGGUC
**SEQ ID NO: 5** hsa-miR-130b-5p MIMAT0004680
   ACUCUUUCCCUGUUGCACUAC
**SEQ ID NO: 6** hsa-miR-130b-3p MIMAT0000691
   CAGUGCAAUGAUGAAAGGGCAU
**SEQ ID NO: 7** hsa-mir-146a_pre MI0000477 
CCGAUGUGUAUCCUCAGCUUUGAGAACUGAAUUCCAUGGGUUGUGUCAGUGUCAGACCUC
UGAAAUUCAGUUCUUCAGCUGGGAUAUCUCUGUCAUCGU
**SEQ ID NO: 8** hsa-miR-146a-5p MIMAT0000449
   UGAGAACUGAAUUCCAUGGGUU
**SEQ ID NO: 9** hsa-miR-146a-3p MIMAT0004608
   CCUCUGAAAUUCAGUUCUUCAG
**SEQ ID NO: 10** hsa-mir-148a_pre MI0000253 
GAGGCAAAGUUCUGAGACACUCCGACUCUGAGUAUGAUAGAAGUCAGUGCACUACAGAACU
UUGUCUC
**SEQ ID NO: 11** hsa-miR-148a-5p MIMAT0004549
   AAAGUUCUGAGACACUCCGACU
**SEQ ID NO: 12** hsa-miR-148a-3p MIMAT0000243
   UCAGUGCACUACAGAACUUUGU
**SEQ ID NO: 13** hsa-mir-181a-1_pre MI0000289 
UGAGUUUGAGGUUGCUUCAGUGAACAUUCAACGCUGUCGGUGAGUUUGGAAUUAAAAU
CAAAACCAUCGACCGUUGAUUGUACCCUAUGGCUAACCAUCAUCUACUCCA
**SEQ ID NO: 14** hsa-mir-181a-2_pre MI0000269 
AGAAGGGCUAUCAGGCCAGCCUUCAGAGGACUCCAAGGAACAUUCAACGCUGUCGGUGAGU
UUGGGAUUUGAAAAACCACUGACCGUUGACUGUACCUUGGGGUCCUUA
**SEQ ID NO: 15** hsa-miR-181a-5p MIMAT0000256
   AACAUUCAACGCUGUCGGUGAGU
**SEQ ID NO: 16** hsa-miR-181a-3p MIMAT0004558
   ACCACUGACCGUUGACUGUACC
**SEQ ID NO: 17** hsa-mir-192_pre MI0000234 
GCCGAGACCGAGUGCACAGGGCUCUGACCUAUGAAUUGACAGCCAGUGCUCUCGUCUCCCC
UCUGGCUGCCAAUUCCAUAGGUCACAGGUAUGUUCGCCUCAAUGCCAGC
**SEQ ID NO: 18** hsa-miR-192-5p MIMAT0000222
   CUGACCUAUGAAUUGACAGCC
**SEQ ID NO: 19** hsa-miR-192-3p MIMAT0004543
   CUGCCAAUUCCAUAGGUCACAG
**SEQ ID NO: 20** >hsa-mir-30a_pre MI0000088 
GCGACUGUAAACAUCCUCGACUGGAAGCUGUGAAGCCACAGAUGGGCUUUCAGUCGGAUG
UUUGCAGCUGC
**SEQ ID NO: 21** >hsa-miR-30a-5p MIMAT0000087
   UGUAAACAUCCUCGACUGGAAG
**SEQ ID NO: 22** >hsa-miR-30a-3p MIMAT0000088
   CUUUCAGUCGGAUGUUUGCAGC

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Identification of circulating microRNAs linked to cognitive function in healthy humans

In context of the invention young healthy individuals were recruited (age: 25.95 ± 5.1 years, n =132) that were subjected to a battery of six different cognitive tests (Budde et al., 2018). Blood was collected from all participants at the time of memory testing and smallRNAome sequencing was conducted **(****Fig 1A****).** To identify microRNAs that correlate with cognition the inventors first calculated for each of the 132 individuals a composite cognitive score (weighted cognitive performance) based on factor analysis which confirmed the expected variability in cognitive function **(****Fig 1B****).** Next, the inventors performed a weighted microRNA co-expression analysis to identify expression-modules **(not shown)**. The inventors then asked if any of these microRNA modules correlate with the weighted cognitive performance and detected 3 modules that were significantly linked to cognition. The turquoise and blue modules were negatively correlated with cognitive performance while the brown module showed a positive correlation with cognition **(****Fig 1C****)**. Next, the inventors performed a GO-term analysis of the confirmed mRNA targets of the 3 microRNA modules and observed that changes in their expression are generally linked to biological functions that reflect aging and age-related processes that are also known to play a role in cognitive function and decline **(****Fig. 1D****).** These data are in line with the view that aging is the major risk factor for cognitive decline and dementia.

### Example 2: Identification of circulating microRNAs in longitudinal mouse model for age-associated memory decline

Age-associated memory decline is a well-established and highly reproducible phenotype in rodents and in humans (Duzel et al., 2016). The inventors hypothesized that analyzing mice from 12 until 16.5 month of age should allow the inventors to detect cognitive decline in a longitudinal setting. To avoid any effect associated with the first exposure to the water maze paradigm, 12-months old mice were habituated to the training procedure. Subsequently, all mice were subjected to water maze training followed by a memory test and blood collection every 1.5 months **(****Fig 2A****).** Importantly, the longitudinal collection of blood from the orbital sinus of mice did not affect vision in the water maze paradigm, which is a pre-requisite to perform the test **(not shown)**. The escape latency during the training procedure - a common measure of spatial learning ability - was significantly impaired when comparing mice at 16.5-months of age to their performance at 13.5 or 15 months of age **(****Fig 2B**). For a more sensitive analysis the inventors employed a modified version of the MUST-C algorithm to measure the different spatial strategies that represent hippocampus-dependent and cognitively demanding as well as hippocampus-independent strategies (Illouz et al., 2016). The results indicate that specifically between 15 and 16.5 months of age, mice adopt search strategies indicative of impaired cognitive ability (**Fig 2C**). Thus, there was a notable reduction of "direct", "corrected" and "short chaining" search strategies that reflect hippocampus-dependent cognitive functioning. On the other hand, strategies independent of hippocampus-dependent cognitive ability such as "long chaining", "circling" and "random" increased at 16.5 months of age (**Fig 2C**). In line with this observation the cognitive score, which was calculated on the basis of the different search strategies during the training procedure, was significantly reduced when comparing mice at 16.5 months to their performance at either 13.5 or 15 months of age (**Fig 2D**). Similar data were obtained in the probe test to assay memory retrieval **(not shown)**. Since all mice were able to find the platform rapidly in a visually cued test performed at 12 and 16.5 months of age **(not shown)** and also the analysis of swimming speed did not reveal any significant differences, these data show that age-associated defects in spatial reference learning can reliably be detected in mice between 13.5 and 16.5 months of age. Next, the inventors subjected blood-derived RNA collected from all experimental groups of mice to small RNA sequencing. The corresponding data was fit to a likelihood ratio test model and adjusted for hidden confounding factors to detect microRNAs that were differentially expressed during the aging process using the expression at 12 months of age as a reference point. The inventors also controlled for microRNAs that were potentially affected by the training procedure **(not shown)**. Thereby, the inventors detected 55 differentially expressed circulating microRNAs during aging **(****Fig 2E****)**. To identify amongst these 55 microRNAs the ones that would inform best about cognitive status and cognitive decline, the inventors employed the sequencing and cognitive data to perform an unbiased multivariate microRNA feature selection.

First, the inventors constructed a composite score from the different water maze features using a principle component (PCA) analysis. To identify microRNAs that inform about memory performance, the inventors subjected these scores along with the expression data of the 55 microRNAs to 3 independent methods for feature selection, namely random forest approaches using multivariate bootstrapping or multivariate leave one out cross validation (Looc) and a support vector machine approach. All 3 methods identified a common 7-microRNA signature linked to age-associated memory performance consisting of let-7b-5p, micro-181a-5p, micro-146a-5p, micro-192-5p, micro-30a-3p, micro-148a-3p and micro-130b-3p (**Fig 2F**). All 7 microRNAs were part of either the brown or blue co-expression modules that were significantly correlated to cognitive performance in healthy humans (Fig **2G****).** These data suggest that the 7 microRNAs are bona fide candidates for circulating biomarkers of cognitive reserve. Encouraged by these findings, the inventors decided to further validate the significance of the 7 microRNAs using another human dataset. Recent GWAS studies identified 709 genes that are associated with general cognitive function in healthy individuals (Marioni et al., 2018). When the inventors asked if target genes of any of the 7 microRNAs would be enriched amongst the 709 genes linked to cognition in humans, the inventors observed that targets of microRNA-181a-5p, microRNA146a-5p and microRNA148a-3p were significantly overrepresented **(****Fig 2H****).** Further analysis suggested that the targets of these 3 microRNAs regulate cellular processes linked to inflammation, vascular and neuronal plasticity and thus represent multiple key processes of cognitive function that are de-regulated during cognitive decline and age-associated dementia **(****Fig 2I****).**

### Example 3: Elevated levels of the 3-microRNA signature are linked to impaired neuronal integrity.

The inventors decided to provide further experimental evidence for the role of the 3 microRNAs in cognitive function. First, the inventors tested if the 3-microRNA signature would indeed help to detect differences in cognitive function in this longitudinal mouse model for age-associated memory decline. Therefore, the inventors devised a statistical framework to test the co-expression of the 3 microRNAs using its eigen-expression, which represents a solid method to decompose gene-expression data into a singular value based on linear transformation (Alter et al., 2000). The eigen-expression of the 3-microRNA signature significantly increased in aging mice between 13.5 and 15 month of age and plateaued at 16.5 months of age **(****Fig 3A****).** Since significant learning impairment was observed only upon 16.5 months of age **(****Fig 3B****;** See also Fig 2), these data indicate that increased expression of the 3-microRNA signature precedes detectable memory impairment in aging mice. The 3 microRNAs of the identified signature are also highly expressed in the brain (Ludwig et al., 2016) and the fact that they were also linked to brain-related processes prompted the inventors to test their role in the brain directly. To this end, the inventors performed small RNA sequencing of the hippocampal sub-regions CA1, CA3 and dentate gyrus (DG) and the anterior cingulate cortex (ACC) isolated from 3 and 16.5 months old mice **(not shown).** Similar to the data obtained in blood samples, expression of the 3-microRNA signature was significantly increased in the brains of cognitively impaired 16.5-month-old mice **(****Fig 3C****).** These data support that altered blood levels of the 3 microRNAs may inform about relevant patho-mechanisms in the brain.

The inventors decided to test the impact of the 3 microRNAs in relevant cell types by increasing their levels via lipid nanoparticles containing the corresponding mimic oligonucleotides. Based on the relative enrichment in the different neural cells, the inventors administered micro-181a-5p and micro-148-3p mimics to hippocampal neuronal and microRNA-146a-5p to immortalized microglia cultures and subsequently performed RNA-sequencing. The inventors observed substantial changes in gene-expression **(****Fig 3E****).** Gene ontology analysis revealed that down-regulated genes were linked to neuronal plasticity and learning and memory in case of micro-181a-5p and micro-148-3p mimics **(****Fig 3F****).** Increasing miR146a-5p levels in the microglia culture causes the down-regulation of genes linked to DNA-repair and protein folding **(****Fig 3F****).** When the inventors analyzed the up-regulated genes, the inventors observed that elevated levels of microRNA-146-5p increase pathways linked to cellular stress and inflammation **(****Fig 3F****)** and could furthermore confirm that increased levels of microRNA-146a-5p in microglia cells lead to the up-regulation of the pro-inflammatory cytokines IL-ibeta, IL6 and TNFalpha, while the anti-inflammatory cytokine IL-10 was decreased **(****Fig 3G****).** Increased levels of microRNA-181a-5p and microRNA-146a-5p were associated with pathways representing neuronal apoptosis and cell death, hypoxia and remodeling of the extracellular matrix **(****Fig 3F****).** In sum, these data support the hypothesis that increased levels of the 3 microRNAs represent multiple mechanisms indicating a low cognitive reserve and a risk to develop cognitive decline. To further substantialize this finding, the inventors compared this gene-expression data to transcriptome and proteome datasets previously linked to neurodegenerative diseases. Interestingly, the genes deregulated in response to microRNA-146a-5p over-expression significantly overlapped with immune response genes recently reported by eQTL analysis (Gioneska et al., 2015) confirming a role of microRNA-146a-5p in neuroinflammation **(****Fig 3H****).** To a lesser extent this was also true for microRNA-148a-3p regulated genes, while no overlap of the eQTL data was found to genes regulated by microRNA-181a-5p, which is in line with the data linking microRNA-148a-3p and specifically microRNA-181a-5p to neuronal processes **(****Fig. 3H****).** The inventors also compared the transcriptomic findings to gene-expression datasets from CK-p25 mice, a mouse model for AD-like neurodegeneration (Fischer et al., 2005) as well as gene-expression and proteome data from human AD patients. The inventors observed that the genes and proteins down-regulated in AD patients strongly overlapped with the down-regulated genes observed in response to microRNA-148a-3p and especially microRNA-181a-5p **(****Fig. 3H****).**

To provide further evidence for this interpretation, the inventors decided to directly analyze the role of the 3 microRNAs signature in synaptic plasticity. Primary hippocampal mouse cultures that contain neuronal and glia cells were treated with a mixture of mimic oligonucleotides representing the 3 microRNA-signature (3-miR-mix). **(****Fig 4A****).** The inventors first analyzed the number of synapses via STED microscopy to detect colocalization of the pre- and postsynaptic marker proteins Synaptophysin 1 (Syphi) and post-synaptic density protein 95 (PSD-95). Delivery of the 3-miR-mix reduced the number of synapses using 2 independent quantification methods **(****Fig 4B****).** In line with this observation, administration of the 3-miR-mix led to a significant reduction in the number of dendritic spines **(****Fig 4C****).** Next, the inventors decided to test if the observed structural alterations would translate into altered neuronal network plasticity. To this end, primary hippocampal cultures were grown on microelectrode array (MEA) plates to measure spontaneous extracellular potentials. Cultures were treated with the 3-miR-mix or scramble RNA and spontaneous activity was recorded for 24h (every 3 hours for 10 min). Administration of 3-miR-mix led to aberrant activity. Namely, the mean firing rate, number of bursts and network bursts were all severely impaired **(****Fig 4D****),** an effect that was observed across the entire 24h of recoding **(****Fig 4E****).** Taken together, these data show that increased expression of the 3-microRNA signature impairs neural plasticity and provides further evidence that the analysis of this signature in blood might inform about mechanisms linked to cognitive function.

### Example 4: A 3-microRNA signature informs about cognitive status

The above-described findings encouraged the inventors to test if the 3-microRNA signature could help to detect alterations in cognitive function in humans. In a first approach, the inventors analyzed the expression of the 3-microRNA signature in individuals of different age-groups in a cross-sectional setting. Similar to the data obtained in mice, the inventors find evidence that the signature might increase in blood prior to the detection of significant cognitive impairment **(not shown).** Encouraged by these findings obtained from healthy humans, the inventors decided to further test the performance of the 3-microRNA signature in cognitive diseases. First, the inventors analyzed a previously published small-RNA dataset (Kayano et al., 2016) obtained from plasma samples that were collected from patients suffering from mild cognitive impairment (MCI).

The 3-microRNA signature was significantly increased in MCI patients when compared to age-matched healthy individuals **(****Fig 5A****).** The inventors further found that the expression of the 3-microRNA signature was significantly increased in MCI patients **(****Fig 5B****).** Interestingly, data on the CSF levels of Aβ42/40 and phospho-Tau were available for most of the analyzed control and MCI patients of the DELCODE study. When the inventors compared the ability to distinguish the same control individuals from MCI patients via the present 3-microRNA signature in blood to the analysis of Aβ42/40 ratio measured in CSF, the 3-microRNA signature performed equally good, while levels of phospho-Tau did not yet reveal significant changes **(see** **Figure 8****)**. Although the 3-microRNA signature significantly differed amongst controls to MCI patients, the expression was rather variable at the individual level **(****Fig. 5A, B****)**. Therefore, the inventors subjected the data to an unbiased hierarchical clustering analysis and observed two main cluster within the MCI patients, representing either patients with low (low expression cluster) or high expression levels (high expression cluster) of the 3-microRNA signature **(****Fig 5C****).** The inventors further observed that specifically those MCI patients that were part of the "high expression cluster" showed a significant negative correlation of the 3-microRNA signature to cognitive function **(****Fig 5D****).** These data further support that high circulating levels of the 3-microRNA signature may indicate a low cognitive reserve and higher risk for cognitive decline. The inventors were able to directly test this hypothesis - at least in part - in MCI patients of the DELCODE study, since follow-up phenotypic data was available for some - but not for all - of the individuals (n=55). Thus, the inventors asked if high levels of the 3-microRNA signature would be associated with the future conversion from MCI to AD. The inventors were able to compare the expression level of the 3-microRNA signature in MCI patients that converted from MCI to AD within 2 years after blood collection (n= 8) to MCI patients that did not progress to AD within the same time period (n=47). The analysis revealed that the expression of the 3-microRNA signature was significantly higher in MCI patients that converted to AD 2 years after blood collection, when compared to those characterized by stable MCI diagnosis **(****Fig 5E****).** Since the animal experiments suggest that increased blood levels of the 3-microRNA signature are paralleled by corresponding changes in the brain, the inventors asked if the signature would be also increased in the brains of humans suffering from MCI. Since the analysis of post-mortem human brain tissue is often confounded by post-mortem delay, RNA quality and other factors, the inventors decided to analyze data from cerebrospinal fluid (CSF) of living MCI patients. Thus, the inventors compared data from probands that did not suffer from cognitive diseases and age-matched individuals diagnosed with mild cognitive impairment (MCI) as a proxy for the expression of microRNAs in the brain. The analysis of corresponding smallRNA sequencing data revealed that the expression of the 3-microRNA signature was significantly increased in MCI individuals **(****Fig 5F****).** Considering the mechanistic studies the inventors performed in mice, these data suggest that altered levels of the 3-microRNA signature control cellular processes essential for cognitive function also in the human brain. To test this hypothesis more directly, the inventors employed human bioengineered neuronal organoids (BENOs) (Zafeiriou et al., 2020) that were treated with the 3-miR-mix followed by RNA-sequencing. In line with the data obtained in mouse cultures the inventors observed that elevated levels of the 3-microRNA signature induced gene-expression changes and related biological processes linked to cellular stress, while genes representing synaptic function related processes were decreased **(****Fig 5G, H****)**.

Taken together the present findings show that the 3-microRNA signature is a suitable molecular marker to inform about cognitive reserve and help to detect of individuals at risk to develop dementia. In fact, the 3-microRNA signature was consistently de-regulated in a meta-analysis performed on 15 different human and mouse datasets **(not shown)**. Specificity of this observation was further demonstrated by the observation that any random set of 3 microRNAs (1000 random combinations were tested) detected in the present dataset failed to yield any significant effect **(not shown)**. Moreover, when the tested the expression of each individual microRNA of the signature or combinations of two of them across the 15 different mouse and human datasets, the inventors did not obtain homogenous data strengthening the view that the well-curated signature outperforms the analysis of individual microRNAs.

### Example 5: The3-microRNA signature is a target for RNA-therapeutics in dementia

The present finding that the 3-microRNA signature is not only increased in blood but also in the brain of cognitively impaired mice and in CSF of MCI patients suggest that targeting this signature in the CNS might be a suitable approach for RNA-therapeutics. To test this hypothesis, the inventors used lipid nanoparticles containing a mix of miR-inhibitor sequences targeted towards microRNA-181a-5p, microRNA-148a-3p and microRNA-146a-5p (anti-miR mix) and administered these particles to the dorsal hippocampus of 16.5-months old mice (anti-miR-mix group). Mice of the same age (old-control group) and 3-months old young mice (young-control group) that were injected with corresponding scramble oligonucleotides served as control **(****Fig 6A****)**. Five days post-injection mice were subjected to the water maze training to test spatial reference memory. Mice of the old-control group displayed a significantly increased escape latency, when compared to the young-control group indicating impaired spatial reference memory **(****Fig 6B, C****)**. Notably, old mice injected with the anti-miR-mix performed comparable to young mice, suggesting improved hippocampus-dependent memory function **(****Fig 6B, C****)**. The inventors analyzed the search strategies during the training procedure in greater detail and observed that mice of old-control group less efficiently adapted hippocampus-dependent search strategies when compared to young mice and old mice treated with the anti-miR-mix **(****Fig 6D****)**. This effect was significant, when the inventors analyzed the search strategies by calculating a cumulative cognitive score on the 5 days of training **(****Fig 6E****)**. During a probe test, mice of the old-control group displayed a reduced number of platform crossings when compared to the young control group, suggesting impaired memory retrieval **(****Fig 6F****)**. In contrast, old mice injected with the anti-miR-mix performed similar to young mice **(****Fig 6F****)**. These data suggest that an RNA-based therapeutic approach targeted towards the 3-microRNA signature can improve memory function in cognitively impaired old mice. Considering that the 3-microRNA signature was also increased in MCI patients, the inventors decided to test its therapeutic potential also in a disease model for AD. The inventors employed APPPS1 mice, a well-established model for amyloid-deposition that displays hippocampus-dependent memory impairment at 6-8 months of age (Radde et al., 2006) (Agís-Balboa et al., 2017) (Martinez-Hernandez et al., 2017). Therefore, the inventors performed smallRNA sequencing of hippocampal tissue obtained from APPPS1 mice at 4 and 8 months of age, representing time points before and after the onset of detectable memory impairment. This data reveal that the 3-microRNA signature is significantly increased already at 4 and also at 8 months of age when comparing APPPS1 to age-matched control mice **(****Fig 6G****)**. Encouraged by this observation, the inventors decided to test if administration of the anti-miR-mix could ameliorate memory impairment in APPPS1 mice. The inventors decided to employ 7 months old APPPS1 mice and injected the anti-miR-mix into the dorsal hippocampus (APP anti-miR-mix group). As control groups the inventors injected age-matched APPPS1 (APP control group) and wild type mice (WT control group) with a mix of corresponding scrambled oligomers. When the inventors subjected mice to the water maze training, the anti-miR-mix group was able to significantly improve the escape latency in APPPS1 mice **(****Fig 6H, I****)** suggesting that the anti-miR-mix ameliorates memory impairment in a mouse model for AD. This effect was also obvious when, the inventors analyzed in detail the different search strategies. Thus, APP mice failed to adapt hippocampus-dependent learning strategies, while APP mice treated with the anti-miR-mix displayed an increase of such strategies similar to wild type control mice **(****Fig 6J****)**. This effect was also highly significant when the inventors analyzed the cumulative score of the search strategies at the 5 days of training **(****Fig 6K****)**. Furthermore, improved memory retrieval during the probe test was observed in anti-miR-mix treated APP mice **(****Fig 6L****)**. In conclusion, these data support the view that targeting the 3-microRNA signature could be a suitable strategy for a biomarker-guided RNA therapy towards dementia.

### Example 6: Targeting the 3-microRNA signature partially reinstates transcriptional homeostasis in disease models

While microRNAs control cellular homeostasis at the level of transcriptional networks, aberrant gene-expression is key hallmark of cognitive diseases including AD (Fischer, 2014b). Thus, the inventors hypothesized that reinstatement of memory function in aged and in APPPS1 mice might - at least in part - be due to the action of the anti-miR-mix on hippocampal gene-expression. To test this, the inventors performed RNA-sequencing from hippocampal tissue of aged and APPPS1-21 mice that received either control RNA or the anti-miR-mix **(****Fig. 7A****).** Wild type littermates treated with control RNA were used as additional control. First the inventors analyzed the data from the experiment employing aged mice and performed Weighted Gene Co-expression Analysis (Langfelder and Horvath, 2008). The inventors identified 29 different co-expression modules in the entire RNA-seq dataset of which 4 represent neuronal cluster. While three of these modules were unaffected among groups, the MEblue module paralleled this behavioral findings and was significantly de-regulated when comparing the young-control to the old-control group, whereas its expression was partially reinstated to the level of the young-control group in response to anti-miR-mix treatment **(****Fig 7B****).** Gene ontology analysis revealed that the genes of the MEblue module represent processes linked to synapse organization and cognition **(****Fig 7C****).** The inventors confirmed the expression of three representative genes, namely LRKK2, Cadm3 and Slc6a11 **(****Fig 7D****).** In the RNA-sequencing data obtained from APPPS1 mice weighted Gene Co-expression Analysis (Langfelder and Horvath, 2008) allowed the inventors to detected 26 different co-expression modules. Of these modules, MElightgreen and MEblue overlapped with neuronal gene set with high significance (**not shown**). However, only MElightgreen module was significantly de-regulated when comparing the WT control group to the APP control group **(****Fig 7G****,),** while its expression was partially normalized to control levels in the APP anti-miR-mix group **(****Fig 7G****).** The genes within the MElightgreen module represent processes linked to synaptic function, similar to the genes for the MEblue module detected in aged mice **(****Fig 7H****).**

Indeed, the inventors confirmed the differential expression in control and APPPS1-21 mice via qPCR for two representative genes namely AFF2/FMR2 that encodes the fragile X mental retardation protein and Hivep3 which encodes the transcription factor kappa-binding protein 1 **(****Fig 7I****).** These data suggest that targeting the 3 microRNAs signature can help to reinstate - at least in part - transcriptional homeostasis in 2 different animal models for cognitive decline.

### REFERENCES

The references are:
Abbott, A., and Dolgin, E. (2016). Leading Alzheimer's theory survives drug failure. Nature 540,15-16.
Agís-Balboa, R.C., Pinhero, P., Rebola, N., Kerimoglu, C., Benito, E., Gertig, M., Bahari-Javan, S., Jain, G., Burkhardt, S., Delalle, I., et al. (2017). Formin 2 links neuropsychiatric phenotypes at young age to an increased risk for dementia.. EMBO J 36, 2815-2828.
Aksoy-Aksel A, Z.F., Schratt G. (2014). MicroRNAs and synaptic plasticity--a mutual relationship. Philos Trans R Soc Lond B Biol Sci 369, pii: 20130515.
Alter, O., Brown, P.O., and Botstein, D. (2000). Singular value decomposition for genome-wide expression data processing and modeling. Proceedings of the National Academy of Sciences of the United States of America 97,10101-10106.
Ansari, A., Maffioletti, E., Milanesi, E., Marizzoni, M., Frisoni, G. B., Blin, O., Richardson, J. C., Bordet, R., Forloni, G., Gennarelli, M., Bocchio-Chiavetto, L., & PharmaCog Consortium (2019). miR-146a and miR-181a are involved in the progression of mild cognitive impairment to Alzheimer's disease. Neurobiology of Aging 82, 102-109.
Banzhaf-Strathmann, J., Benito, E., May, S., Arzberger, T., Tahirovic, S., Kretzschmar, H., Fischer, A., and Edbauer, D. (2014). MicroRNA-125b induces tau hyperphosphorylation and cognitive deficits in Alzheimer's disease. EMBO J 33, 1667-1680.
Beason-Held, L.L., Goh, J.O., An, Y., Kraut, M.A., O'Brien, R.J., Ferrucci, L., and Resnick, S.M. (2013). Changes in brain function occur years before the onset of cognitive impairment. J Neurosci 33, 18008-18014.
Benito, E., Urbanke, E., Barth, J., Halder, R., Capece, V., Jain, G., Burkhardt, S., Navarro, M., Schutz, A.L., Bonn, S., et al. (2015). Reinstating transcriptome plasticity and memory function in mouse models for cognitive decline. J Clin Invest 125, 3572-3584.
Blennow, K. (2017). A Review of Fluid Biomarkers for Alzheimer's Disease: Moving from CSF to Blood.. Neurol Ther 6, 15-24.
Breiman, L. (2001). (2001). "Random forests." Machine learning 45, 5-32.
Budde, M., Anderson-Schmidt, H., Gade, K., Reich-Erkelenz, D., Adorjan, K., Kalman, J.L., Senner, F., Papiol, S., Andlauer, T.F.M., Comes, A.L., et al. (2018). A longitudinal approach to biological psychiatric research: The PsyCourse study. Am J Med Genet B Neuropsychiatr Genet 180, 89-102.
Chen, L., Dong, R., Lu, Y., Zhou, Y., Li, K., Zhang, Z., and Peng, M. (2019). microRNA-146a protects against cognitive decline induced by surgical trauma by suppressing hippocampal neuroinflammation in mice. Brain Behav Immun 24, 188-201.
Condrat, C.E., Thompson, D.C., Barbu, M.G., Bugnar, O.L., Boboc, A., Cretoiu, D., Suciu, N., Cretoiu, S.M., and Voinea, S.C. (2020). miRNAs as Biomarkers in Disease: Latest Findings Regarding Their Role in Diagnosis and Prognosis.. Cells 9, 276.
Cortes, C., and Vapnik, V. (1995). Support-vector networks. Machine learning 20, 273-297.
Davies G, L.M., Harris SE, et al. (2018). Study of 300,486 individuals identifies 148 independent genetic loci influencing general cognitive function. Nat Commun 1, 2098.
Deary, I.J., Corley, J., Gow, A.J., Harris, S.E., Houlihan, L.M., Marioni, R.E., Penke, L., Rafnsson, S.B., and Starr, J.M. (2009). Age-associated cognitive decline. Br Med Bull 92, 135-152.
Dong , H., Li, J., Huang, L., Chen, X., Li, D., Wang, T., Hu, C., Xu, J., Zhang, C., Zen, K., et al. (2015). Serum MicroRNA Profiles Serve as Novel Biomarkers for the Diagnosis of Alzheimer's Disease. Dis Markers, 625659.
Dong W, W.R., Ma LN, Xu BL, Zhang JS, Zhao ZW, Wang YL, Zhang X. (2016). Influence of age-related learning and memory capacity of mice: different effects of a high and low caloric diet. Aging Clin Exp Res 28, 303-311.
Duzel, E., van Praag, H., and Sendtner, M. (2016). Can physical exercise in old age improvememory and hippocampal function? Brain 139, 662-673.
Fischer, A. (2014a). Epigenetic memory: the Lamarckian brain. EMBO J 33, 945-967.
Fischer, A. (2014b). Targeting histone-modifications in Alzheimer's disease. What is the evidence that this is a promising therapeutic avenue? Neuropsychopharmacology 80, 95-012.
Fischer, A., Sananbenesi, F., Pang, P.T., Lu, B., and Tsai, L.H. (2005). Opposing roles of transient and prolonged expression of p25 in synaptic plasticity and hippocampus-dependent memory. Neuron 48, 825-838.
Franceschi, C., Garagnani, P., Morsiani, C., Conte, M., Santoro, A., Grignolio, A., Monti, D., Capri, M., and Salvioli, S. (2018). The Continuum of Aging and Age-Related Diseases: Common Mechanisms but Different Rates.. Front Med (Lausanne) 12, eCollection2018.
Franklin, S.B., Gibson, J.D., Robertson, P.A., Pohlmann, J.T., and Fralish, J.S. (1995). Parallel Analysis: a method for determining significant principal components. Journal of Vegetation Science 1, 102-123.
Galimberti, D., Villa, C., Fenoglio, C., Serpente, M., Ghezzi, L., Cioffi, S.M., Arighi, A., Fumagalli, G., and Scarpini, E. (2014). Circulating miRNAs as potential biomarkers in Alzheimer's disease. J Alzheimers Dis 42, 1261-1267.
Gjoneska, E., Pfenning, A.R., Mathys, H., Quon, G., Kundaje, A., Tsai, L.H., and Kellis, M. (2015). Conserved epigenomic signals in mice and humans reveal immune basis of Alzheimer's disease. Nature 518, 365-369.
Gurtan, A.M., and Sharp, P.A. (2013). The Role of miRNAs in Regulating Gene Expression Networks. J Mol Biol pii, Epub ahead of print.
Hanna, J., Hossain, G.S., and Kocerha, J. (2019). The Potential for microRNA Therapeutics and Clinical Research. Front Genet 16, 478.
Hassenstab, J., Monsell, S.E., Mock, C., Roe, C.M., Cairns, N.J., Morris, J.C., and Kukull, W. (2015). Neuropsychological Markers of Cognitive Decline in Persons With Alzheimer Disease Neuropathology.. Journal of neuropathology and experimental neurology 74, 1086-1092.
Havas, D., Hutter-Paier, B., Ubhi, K., Rockenstein, E., Crailsheim, K., Masliah, E., and Windisch, M. (2011). A longitudinal study of behavioral deficits in an AβPP transgenic mouse model of Alzheimer's disease. J Alzheimers Dis 25, 231-243.
Hayden, K.M., Jones, R.N., Zimmer, C., Plassman, B.L., Browndyke, J.N., Pieper, C., Warren, L.H., and Welsh-Bohmer, K.A. (2011). Factor structure of the National Alzheimer's Coordinating Centers uniform dataset neuropsychological battery: an evaluation of invariance between and within groups over time. Alzheimer Dis Assoc Disord 25, 128-137.
Hébert, S.S., Horre, K., Nicolai, L., Papadopoulou, A.S., Mandemakers, W., Silahtaroglu, A.N., Kauppinen, S., Delacourte, A., and De Strooper, B. (2008). Loss of microRNA cluster miR-29a/b-1 in sporadic Alzheimer's disease correlates with increased BACE1/beta-secretase expression. Proc Natl Acad Sci U S A 205, 6415-6420.
Herrera-Espejo, S., Santos-Zorrozua, B., Álvarez-González, P., Lopez-Lopez, E., and Garcia-Orad, Á. (2019). A Systematic Review of MicroRNA Expression as Biomarker of Late-Onset Alzheimer's Disease. Molecular neurobiology 56, 8376-8391.
Hill, J.M., and Lukiw, W.J. (2016). MicroRNA (miRNA)-Mediated Pathogenetic Signaling in Alzheimer's Disease (AD). Neurochem Res 41, 95-100.
Illouz, T., Madar, R., Louzon, Y., Griffioen, K.J., and Okun, E. (2016). Unraveling cognitive traits using the Morris water maze unbiased strategy classification (MUST-C) algorithm.. Brain Behav Immun 52, 132-144.
Jaber, V.R., Zhao, Y., Sharfman, N.M., Li, W., and Lukiw, W.J. (2019). Addressing Alzheimer's Disease (AD) Neuropathology Using Anti-microRNA (AM) Strategies.. Molecular neurobiology 56, 8101-8108.
Jack, C.R.J., Bennett, D.A., Blennow, K., Carrillo, M.C., Dunn, B., Haeberlein, S.B., Holtzman, D.M., Jagust, W., Jessen, F., Karlawish, J., et al. (2018). NIA-AA Research Framework: Toward a biological definition of Alzheimer's disease. Alzheimers Dement 14, 535-562.
Jain, G., Stuendl, A., Rao, P., Berulava, T., Pena Centeno, T., Kaurani, L., Burkhardt, S., Delalle, I., Kornhuber, J., Hüll, M., et al. (2019). A combined miRNA-piRNA signature to detect Alzheimer's disease.. Translational Psychiatry 9, 250.
Jessen, F., Spottke, A., Boecker, H., Brosseron, F., Buerger, K., Catak, C., Fliessbach, K., Franke, C., Fuentes, M., Heneka, M.T., et al. (2018). Design and first baseline data of the DZNE multicenter observational study on predementia Alzheimer's disease (DELCODE). Alzheimers Res Ther 10, doi: 10.1186/s13195-13017-10314-13192..
Jovičić, A., Roshan, R., Moisoi, N., Pradervand, S., Moser, R., Pillai, B., and Luthi-Carter, R. (2013). Comprehensive expression analyses of neural cell-type-specific miRNAs identify new determinants of the specification and maintenance of neuronal phenotypes. J Neurosci 33, 5127-5137.
Kayano, M., Higaki, S., Satoh, J.I., Matsumoto, K., Matsubara, E., Takikawa, O., and Niida, S. (2016). Plasma microRNA biomarker detection for mild cognitive impairment using differential correlation analysis.. Biomarker research 4, https://doi.org/10.1186/s40364-40016-40076-40361.
Khanna, A., Muthusamy, S., Liang, R., Sarojini, H., and Wang, E. (2011). Gain of survival signaling by down-regulation of three key miRNAs in brain of calorie-restricted mice. Aging, 223-236.
Kumar, S., Vijayan, M., Bhatti, J.S., and Reddy, P.H. (2017). MicroRNAs as Peripheral Biomarkers in Aging and Age-Related Diseases. Prog Mol Biol Transl Sci 146, 47-94.
Laczó, J., Andel, R., Nedelska, Z., Vyhnalek, M., Vlcek, K., Crutch, S., Harrison, J., and Hort, J. (2017). Exploring the contribution of spatial navigation to cognitive functioning in older adults. Neurobiol Aging 51, 67-70.
Langfelder, P., and Horvath, S. (2008). WGCNA: an R package for weighted correlation network analysis. BMC Bioinformatics 29, eCollection.
Leidinger, P., Backes, C., Deutscher, S., Schmitt, K., Muller, S.C., Frese, K., Haas, J., Ruprecht, K., Paul, F., Stahler, C., et al. (2013). A blood based 12-miRNA signature of Alzheimer disease patients. Genome Biol 14, R78.
Lepko, T., Pusch, M., Müller, T., Schulte, D., Ehses, J., Kiebler, M., Hasler, J., Huttner, H. B., Vandenbroucke, R. E., Vandendriessche, C., Modic, M., Martin-Villalba, A., Zhao, S., LLorens-Bobadilla, E., Schneider, A., Fischer, A., Breunig, C. T., Stricker, S. H., Götz, M., & Ninkovic, J. (2018). Choroid plexus-derived miR-204 regulates the number of quiescent neural stem cells in the adult brain. EMBO J 38, e100481.
Li, D., and Mielke, M.M. (2019). An Update on Blood-Based Markers of Alzheimer's Disease Using the SiMoA Platform.. Neurology and therapy 8, 73-82.
Liang H, H.L., Cao J, Zen K, Chen X, Zhang CY. (2012). Regulation of mammalian gene expression by exogenous microRNAs. Wiley Interdiscip Rev RNA 3, 733-742.
Livak, K.J., and Schmittgen, T., D. (2001). Analysis of relative gene expression data using real-time quantitative PCR and the 2- ΔΔCT method. Methods 25, 402-408.
Love, M.I., Huber, W., and Anders, S. (2014). Moderated estimation of fold change and dispersion for RNA-seq data with DESeq2. Genome Biol 15, 550.
Ludwig, N., Leidinger, P., Becker, K., Backes, C., Fehlmann, T., Pallasch, C., Rheinheimer, S., Meder, B., Stähler, C., Meese, E., et al. (2016). Distribution of miRNA expression acrosshuman tissues. Nucleic Acids Res 44, 3865-3877.
Maffioletti, E., Milanesi, E., Ansari, A., Zanetti, O., Galluzzi, S., Geroldi, C., Gennarelli, M., and Bocchio-Chiavetto, L. (2020). miR-146a Plasma Levels Are Not Altered in Alzheimer's Disease but Correlate With Age and Illness Severity. Front Aging Neurosci 17, 366.
Marioni, R.E., Harris, S.E., Zhang, Q., McRae, A.F., Hagenaars, S.P., Hill, W.D., Davies, G., Ritchie, C.W., Gale, C.R., Starr, J.M., et al. (2018). GWAS on family history of Alzheimer's disease. Transl Psychiatry 8, 161.
Martinez-Hernandez, A., Urbanke, H., Gillman, A.L., Lee, J., Ryazanov, S., Agbemenyah, H.Y., Benito, E., Jain, G., Kaurani, L., Grigorian, G., et al. (2017). The diphenylpyrazol compound anle138b blocks AD channels and rescues disease phenotypes in a mouse model for amyloid pathology. EMBO MolMed 10, 32-47.
Maschmeyer, P., Petkau, G., Siracusa, F., Zimmermann, J., Zügel, F., Kühl, A.A., Lehmann, K., Schimmelpfennig, S., Weber, M., Haftmann, C., et al. (2018). elective targeting of pro-inflammatory Thi cells by microRNA-148a-specific antagomirs in vivo. J Autoimmun 89, 41-52.
Means, L.W., Higgins, J.L., and Fernandez, T.J. (1993). Mid-life onset of dietary restriction extends life and prolongs cognitive functioning. Physiol Behav 54, 503-508.
Mitchell, P.S., Parkin, R.K., Kroh, E.M., Fritz, B.R., Wyman, S.K., Pogosova-Agadjanyan, E.L., Peterson, A., Noteboom, J., O'Briant, K.C., Allen, A., et al. (2008). Circulating microRNAs as stable blood-based markers for cancer detection.. Proceedings of the National Academy of Sciences of the United States of America 105, 0513-10518.
Mitjans, M., Seidel, J., Begemann, M., Bockhop, F., Moya-Higueras, J., Bansal, V., Wesolowski, J., Seelbach, A., Ibáñez, M.I., Kovacevic, F., et al. (2018). Violent aggression predicted by multiple pre-adult environmental hits. Mol Psychiatry, Epub ahead of print.
Molinuevo, J.L., Ayton, S., Batrla, R., Bednar, M.M., Bittner, T., Cummings, J., Fagan, A.M., Hampel, H., Mielke, M.M., Mikulskis, A., et al. (2018). Current state of Alzheimer's fluid biomarkers. Acta Neuropathol 136, 821-853.
Moon, J.M., Xu, L., and Giffard, R.G. (2013). Inhibition of microRNA-181 reduces forebrain ischemia-induced neuronal loss.. J Cereb Blood Flow Metab 33, 1976-1982.
Nagaraj, S., Laskowska-Kaszub, K., D 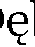 bski, K.J., Wojsiat, J., D 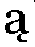 browski, M., Gabryelewicz, T., Kuznicki, J., and Wojda, U. (2017). Profile of 6 microRNA in blood plasma distinguish early stage Alzheimer's disease patients from non-demented subjects. Oncotarget 8, 16122-16143.
Olsson, B., Lautner, R., Andreasson, U., Öhrfelt, A., Portelius, E., Bjerke, M., Hölttä, M., Rosen, C., Olsson, C., Strobel, G., et al. (2016). CSF and blood biomarkers for the diagnosis of Alzheimer's disease: a systematic review and meta-analysis.. The Lancet Neurology 15.
Outlook, N.M. (2019). Delivering the promise of RNA therapeutics. Nature Medicine 25, 1321-1321.
Ouyang, Y.B., Lu, Y., Yue, S., Xu, L.J., Xiong, X.X., White, R.E., Sun, X., and Giffard, R.G. (2012). miR-181 regulates GRP78 and influences outcome from cerebral ischemia in vitro and in vivo. Neurobiol Dis 45, 555-563.
Park, D.C., Lautenschlager, G., Hedden, T., Davidson, N.S., Smith, A.D., and Smith, P.K. (2002). Models of visuospatial and verbal memory across the adult life span. Psychology and aging, . Psychol Aging 17, 299-320.
Peleg, S., Sananbenesi, F., Zovoilis, A., Burkhardt, S., Bahari-Java, S., Agis-Balboa, R.C., Cota, P., Wittnam, J., Gogul-Doering, A., Opitz, L., et al. (2010). Altered histone acetylation is associated with age-dependent memory impairment in mice. Science 328, 753-756.
Ping, L., Duong, D.M., Yin, L., Gearing, M., Lah, J.J., Levey, A.I., and Seyfried, N.T. (2018). Global quantitative analysis of the human brain proteome in Alzheimer's and Parkinson's Disease. Scientific data 5, 180036.
Plaisier SB, T.R., Wong JA, Graeber TG. (2010). aRnk-rank hypergeometric overlap: identification of statistically significant overlap between gene-expression signatures. Nucleic Acids Res 38, e169.
Preische, O., Schultz, S.A., Apel, A., Kuhle, J., Kaeser, S.A., Barro, C., Gräber, S., Kuder-Buletta, E., LaFougere, C., Laske, C., et al. (2019). Dominantly Inherited Alzheimer Network. Serum neurofilament dynamics predicts neurodegeneration and clinical progression in presymptomatic Alzheimer's disease. Nat Med 25, 277-283.
Radde, R., Bolmont, T., Kaeser, S.A., Coomaraswamy, J., Lindau, D., Stoltze, L., Calhoun, M.E., Jäggi, F., Wolburg, H., Gengler, S., et al. (2006). Abeta42-driven cerebral amyloidosis in transgenic mice reveals early and robust pathology. EMBO Rep 7, 940-946.
Rao, P., Benito, E., and Fischer, A. (2013). MicroRNAs as biomarkers for CNS disease. Front Mol Neurosci 26, eCollection 2013.
Rodriguez-Ortiz, C.J., Baglietto-Vargas, D., Martinez-Coria, H., LaFerla, F.M., and Kitazawa, M. (2014). Upregulation of miR-181 decreases c-Fos and SIRT-1 in the hippocampus of 3xTg-AD mice. J Alzheimers Dis 42, 1229-1238.
Roovers, J., De Jonghe, P., and Weckhuysen , S. (2018). The therapeutic potential of RNA regulation in neurological disorders. Expert Opin Ther Target 22, 1017-1028.
Roy, B., Yoshino, Y., Allen, L., Prall, K., Schell, G., and Dwivedi, Y. (2020). Exploiting Circulating MicroRNAs as Biomarkers in Psychiatric Disorders.. Molecular diagnosis & therapy 24, 279-298.
Rupaimoole, R., and Slack, F., J. (2017). MicroRNA therapeutics: towards a new era for the management of cancer and other diseases.. Nat Rev Drug Discov 16, 203-222.
Saba, R., Sorensen, D.L., and Booth, S.A. (2014). MicroRNA-146a: A Dominant, Negative Regulator of the Innate Immune Response. Front Immunol 21, eCollection 2014.
Saba, R., Störchel, P.H., Aksoy-Aksel, A., Kepura, F., Lippi, G., Plant, T.D., and Schratt, G.M. (2012). Dopamine-regulated microRNA MiR-181a controls GluA2 surface expression in hippocampal neurons. Mol Cell Biol 32, 619-632.
Salta, E., and De Strooper, B. (2017). Noncoding RNAs in neurodegeneration. Nat Rev Neurosci 18, 627-640.
Sambandan, S., Akbalik, G., Kochen, L., Rinne, J., Kahlstatt, J., Glock, C., Tushev, G., Alvarez-Castelao, B., Heckel, A., and Schuman, E.M. (2017). ctivity-dependent spatially localized miRNA maturation in neuronal dendrites. Science 355, 634-637.
Schaie, K.W. (1993). The Seattle Longitudinal Study: a thirty-five-year inquiry of adult intellectual development.. Z Gerontol 26, 129-137.
Schneider, L.S., Mangialasche, F., Andreasen, N., Feldman, H., Giacobini, E., Jones, R., Mantua, V., Mecocci, P., Pani, L., Winblad, B., et al. (2014). Clinical trials and late-stage drug development for Alzheimer's disease: an appraisal from 1984 to 2014. J Intern Med 275, 151-283.
Sperling, R., Mormino, E., and Johnson, K. (2014). The evolution of preclinical Alzheimer's disease: implications for prevention trials. Neuron 84, 608-622.
Stepniak, B., Kästner, A., Poggi, G., Mitjans, M., Begemann, M., Hartmann, A., Van der Auwera, S., Sananbenesi, F., Krueger-Burg, D., Matuszko, G., et al. (2015). Accumulated common variants in the broader fragile X gene family modulate autistic phenotypes. EMBO Mol Med 7, 1565-1579.
Stern, Y. (2009). Cognitive reserve. Neuropsychologia. Neuropsychologia 47, 2015-2028.
Stern, Y. (2012). Cognitive reserve in ageing and Alzheimer's disease.. Lancet Neurol 11, 1006-1012.
Stilling, R.M., Benito, E., Gertig, M., Barth, J., Capece, V., Burkhardt, S., Bonn, S., and Fischer, A. (2014). De-regulation of gene expression and alternative splicing affects distinct cellular pathways in the aging hippocampus. Front Cell Neurosci 8, doi: 10.3389/fncel.2014.00373.
Swarbrick, S., Wragg, N., Ghosh, S., and Stolzing, A. (2019). Systematic Review of miRNA as Biomarkers in Alzheimer's Disease.. Molecular neurobiology 56.
Vliegenthart, A.D.B., Berends, C., Potter, C.M.J., Kersaudy-Kerhoas, M., and Dear, J.W. (2017). MicroRNA-122 can be measured in capillary blood which facilitates point-of-care testing for drug-induced liver injury. Br J Clin Pharmacol 83, 2027-2033.
Wang, G., Huang, Y., Wang, L.L., Zhang, Y.F., Xu, J., Zhou, Y., Lourenco, G.F., Zhang, B., Wang, Y., Ren, R.J., et al. (2016). MicroRNA-146a suppresses ROCK1 allowing hyperphosphorylation of tau in Alzheimer's disease. Sci Rep 25, 26697.
Witwer, K.W. (2015). Circulating MicroRNA Biomarker Studies: Pitfalls and Potential Solutions. Clin Chem 61, 56-63.
Xu, L.J., Ouyang, Y.B., Xiong, X., Stary, C.M., and Giffard, R.G. (2015). Post-stroke treatment with miR-181 antagomir reduces injury and improves long-term behavioral recovery in mice after focal cerebral ischemia.. Exp Neurol 26, 1-7.
Zafeiriou, M.A., Guobin, B., Hudson, J., Halder, R., Blenkle, A., Schreiber, M.A., Fischer, A., Schild, D., and Zimmermann, W.A. (2020). Developmental GABA polarity switch and neuronal plasticity in Bioengineered Neuronal Organoids. Nature Communications in press.
Zampetaki, A., Willeit, P., Drozdov, I., Kiechl, S., and Mayr, M. (2012). Profiling of circulating microRNAs: from single biomarkers to re-wired networks.. Cardiovascular research 93, 555-562.
Zhang, Y., Kim, M.S., Jia, B., Yan, J., Zuniga-Hertz, J.P., Han, C., and Cai, D. (2017). Hypothalamic stem cells control ageing speed partly through exosomal miRNAs. Nature 548, 52-57.
Zheng, W., Yao, L., Teng, J., Yan, C., Qin, P., Liu, G., and Chen, W. (2018). Lateral Flow Test for Visual Detection of Multiple MicroRNAs. Sens Actuators B Chem 1, 320-326.
Zovoilis, A., Agbemenyah, H.Y., Agis-Balboa, R.C., Stilling, R.M., Edbauer, D., Rao, P., Farinelli, L., Delalle, I., Schmitt, A., Falkai, P., et al. (2011). microRNA-34c is a novel target to treat dementias. EMBO J 30, 4299-4308.

## Claims

1. **A method for detecting or diagnosing a neurological and** / **or neuropsychological condition, or determining a risk of developing a neurological and** / **or neuropsychological condition, in a subject,** the method comprising the steps of:
(i) Providing a biological sample of the subject;
(ii) Detecting at least the presence of the biomarkers microRNA-181a-5p, microRNA146a-5p and microRNA148a-3p, and optionally of a further biomarker selected from let-7b-5p, miR-130b-3p, miR-192-5p and miR-30a-3p, in the biological sample; and
wherein the detection of the biomarkers in the biological sample indicates the presence of the neurological and / or neuropsychological condition, or a risk of developing the neurological and / or neuropsychological condition, in the subject.

2. The method of claim 1, which is not a surgical method, preferably is a non-invasive method, preferably which is an ex-vivo or in-vitro method.

3. The method of claim 1 or 2, wherein the subject is a mammal, preferably a human, more preferably wherein the subject has no immediate symptom, or mild symptoms, of the neurological and/or neuropsychological condition.

4. The method of any one of claims 1 to 3, wherein the biological sample is selected from the group consisting of a fluid sample, preferably a fluid sample containing micro RNA, such as a cerebrospinal sample, more preferably a blood sample, such as a whole blood, serum sample or plasma sample, or a fraction thereof such as a cell containing fraction or an extracellular vesicle (or exosomes) containing sample.

5. The method of any one of claims 1 to 4, wherein step (ii) includes a step of quantifying the level of the biomarker, and wherein a differential level, preferably increased level, of the biomarker compared to a control or reference indicates the presence of the neurological and / or neuropsychological condition, or a risk of developing the neurological and / or neuropsychological condition, in the subject.

6. The method of any one of claims 1 to 5, wherein step (ii) is carried out by PCR, quantitative PCR (qPCR), RT-qPCR, droplet digital PCR, next generation sequencing or a hybridization based method such as Southern blot and/or lateral flow or microfluidic based assays.

7. The method of any one of claims 1 to 6, wherein the neurological and / or neuropsychological condition is associated with inflammatory processes and/or reduced neuronal plasticity.

8. The method of any one of claims 1 to 7, wherein the neurological and / or neuropsychological condition is associated with a reduced learning capability of the subject compared to a healthy subject.

9. The method of any one of claims 1 to 8, wherein the neurological and / or neuropsychological condition is associated with memory decline of the subject, preferably associated with age-associated memory decline, for example wherein the neurological and / or neuropsychological condition is a cognitive disorder, preferably selected from a form of dementia, such as Alzheimer's Disease (AD).

10. The method of any one of claims 1 to 9, wherein the method is for determining a risk of the subject to develop the neurological and / or neuropsychological condition.

11. **A micro RNA (miR) antagonist composition for use in the prevention or treatment of a neurological and** / **or neuropsychological condition** in a subject, wherein the micro RNA antagonist composition comprises at least one miR antagonist of at least one miR selected from miR-146a-5p, miR-148a-3p, and miR-181a-5p.

12. The miR antagonist composition for use of claim 11, which comprises miR antagonists against microRNA-181a-5p, microRNA146a-5p and microRNA148a-3p.

13. The miR antagonist composition for use of claim 11 or 12, which is an antisense nucleic acid comprising a sequence which is antisense or hybridizes to the at least one miR.

14. The miR antagonist composition for use of any one of claims 11 to 13, wherein subject is a mammal, preferably a human, more preferably wherein the subject has no immediate symptom, or mild symptoms, of the neurological and / or neuropsychological condition, but which was diagnosed of having a risk of developing the neurological and / or neuropsychological condition, or which already developed mild or severe symptoms of the neurological and / or neuropsychological condition.

15. **A diagnostic kit for use in a method for detecting or diagnosing a neurological and / or neuropsychological condition, or determining a risk of developing a neurological and / or neuropsychological condition, in a subject,** comprising primers or probes specific for the detection and/or quantification of the at least the biomarkers microRNA-181a-5p, microRNA146a-5p and microRNA148a-3p.
